# EUROPEAN PATENT SPECIFICATION

(11) **EP 3 833 412 B1**
(45) Date of publication and mention of the grant of the patent: **29.10.2025**
(21) Application number: 18925742.1
(22) Date of filing: 10.07.2018
(51) Int. Cl.: A61M 15/00, G06M 1/08

(54) **PRIMING MEMBER FOR AN INHALER FOR INHALING A DOSE OF A POWDER**
AUSLÖSEELEMENT FÜR EINEN INHALATOR ZUM INHALIEREN EINER PULVERDOSIS
ÉLÉMENT D'AMORÇAGE POUR UN INHALATEUR DESTINÉ À L'INHALATION D'UNE DOSE DE POUDRE

(43) Date of publication of application: 16.06.2021
(73) Proprietor: Mylan Inc., Canonsburg, PA 15317 (US)
(72) Inventor: HOLROYD, Michael, Dublin (IE); BOWMAN, Nicolas John, Cambridge CB21 6GP (GB)
(74) Representative: Peters, Sebastian Martinus
(86) International application number: PCT/US2018/041424
(87) International publication number: WO 2020/013809

(56) References cited:
- EP-A1- 3 042 681
- WO-A1-2013/175177
- WO-A2-2005/002654
- US-A1- 2007 175 474
- US-A1- 2007 181 123
- US-A1- 2007 181 123
- US-A1- 2009 293 874
- US-A1- 2010 083 962
- US-A1- 2011 226 244
- US-A1- 2015 174 346

## Description

### BACKGROUND

Development of inhalation technology for drug delivery has contributed immensely in treating various intrapulmonary and extrapulmonary diseases. This is supported by the unique geometry of the lungs such as a large surface area, thin alveolar epithelial lining, high vascularization, and avoidance of first-pass metabolism. Numerous inhalation delivery systems have been developed and studied to treat lung diseases such as asthma, chronic obstructive pulmonary disease (COPD), and other pulmonary infections. Among them, three approaches, that is, nebulizers, pressurized metered-dose inhalers (pMDIs), and dry powder inhalers (DPI), are extensively scrutinized for the treatment of several lung diseases and pathological conditions. Utilization of nebulizers requires bulky compressors or a source of compressed air, while pMDIs have limitations such as sedimentation, crystal growth, and selection of appropriate propellant and they emit dosages at high velocity, which build deposition in the oropharynx, most commonly where they are swallowed and enhance the risk of systemic absorption. DPIs were introduced to cure some of the weaknesses associated with nebulizers and pMDIs.

A wide variety of devices are known for dispensing doses of medicament in the form of powder for inhalation. Devices are known which contain a store of powdered medicament from which individual doses are metered as required. Devices are also known which include carriers having a plurality of pockets containing respective doses of powder. These carriers are typically in the form of blister-packs. All of these devices face problems of providing reliable, repeatable and accurate inhaled amounts of powder.

There are problems in ensuring that all of a dispensed dose of powder is entrained into the airstream for inhalation. There are other problems in providing repeatable and consistent release of powder into the inhalation airstream as desired, problems with over or under delivery of medication. For example, when an inhaling device is misused by not actuating the lever fully or by not conforming with other instructions for use, the counters may decrement prior to a dose having been made available to the user and the user may be missing needed medication. These mistakes can be repeated for multiple actuations.

Therefore, it would be beneficial to provide an inhaling device that incorporates a design feature that would minimize the incidence of counter miscounting and/or misindexing.

EP 3042681 A1 discloses an inhaler comprising an outer body including a mouth portion through which a drug is inhaled, a cartidge portion in a disc shape rotatably supported inside the outer body and including a plurality of housing portions that seals and houses the drug, and a nozzle portion provided inside the outer body, formed so as to be able to open the housing portion by being driven and fluidly connects an inside of the housing portion and the mouth portion by opening the housing portion.

WO 2013/175177 A1 discloses an inhaler comprising a housing to receive a strip having a plurality of blisters, each blister having a puncturable lid and containing a dose medicament for inhalation by a user. The inhaler has a mouthpiece mounted to the housing and through which a dose of medicament is inhaled by the user, a cap that covers the mouthpiece in a closed position and an actuating element. The cap and the mounting element are mounted coaxially for rotation about a first axis to sequentially move each blister into alignment with a blister piercong member upon rotation by the cap by a user about said first axis into an open position in which the mouthpiece is revealed. The blister piercing member is mounted for rotation about a second axis and cooperates with the actuating element so that the blister piercing member pivots about said second axis in response to rotation of the cap about the first axis to puncture the lid of an aligned blister. An airflow through the blister is generated to entrain the dose contained therein and carry it via the mouthpiece into the user's airway when the user inmhales through the mouthpiece.

US 2010/083962 A1 discloses a dispenser for pulverulent substances, in particular medicaments, from a blister pack, dome-shaped cavities of which that are disposed on a base layer can, by movement in a dispenser housing, be brought step by step into an emptying position, in which they can be opened by means of a needle and can be emptied by a suction air streamleading top a mouthpiece. To achieve optimum emptying of the cavitiesd, it is proposed that the needle passes crosswise through both lateral walls of the dome of the cavities in order to form a through path to the mouthpiece for suction air.

US 2009/293874 A1 discloses a delivery device, e.g. an inhaler, which comprises a rotatable metering member adapted to dispense a measured amount of material, a material delivery passage and a material delivery orifice and at least one actuating member adapted to move the metering member from a material retaining position to a material dispensing position. In a magazine comprising a plurality of metering members, the actuator and metering members may operate in a radial direction. A plurality of rotatable magazines may be utilized in the delivery device, which devide may used to deliver a single or a combination therapy.

US 2011/226244 A1 discloses dry powder inhalers with an inhaler body defining an enclosed cavity space and at least one of an endless strip having opposing primary surfaces, the strip comprising a plurality of spaced apart blisters or dose containers holding dry powder medicament. The inhaler also has an inhalation exit flow path in the inhaler body in communication with at least one blister or at least one dose container held by the strip in an dispensing position an a piercer configured to release dry powder medicament from the blister or dose container in the dispensing position.

US 2007/181123 A1 discloses a device for dispensing individual doses of powder from respective pockets of a disc-shaped carrier by outwardly rupturing a lidding foil by means of pressure on an opposite side surface, the device providing individual respective deaggregation flow path for each pocket, split airsteams allowing improved entrainment of powder, a cam mechanism for outwardly rupturing the pockets, an indexing mechanism linked to the cam mechanism and a dose counter.

### SUMMARY OF THE INVENTION

The present invention provides a priming member for an inhaler as defined in claim 1, and an inhaler for inhaling a dose of powder as defined in claim 7. Further preferred embodiments of the present invention are defined in the dependent claims.

### SUMMARY OF THE DISCLOSURE

Reference will now be made to various exemplary embodiments or aspects of the present disclosure or application. Embodiments or aspects disclosed herein which do not fall under the scope of the appended claims do not form part of the present invention, but are useful for understanding the principles of the invention. The scope of the present invention is defined by the appended claims.

The present application provides a priming member for an inhaler. The priming member comprises a cantilever blade configured to engage a chassis of the inhaler. The cantilever blade has a proximal portion, a body and a distal portion, the body being disposed between the proximal portion and the distal portion of the cantilever blade. The cantilever blade has a flexible portion extending with the proximal portion of the cantilever blade to allow movement of the cantilever blade relative to the priming member. The flexible portion of the cantilever blade is a hinge flexure allowing the cantilever blade to move in an upward and downward direction relative to the chassis of the inhaling device.

The cantilever blade is adapted to pass over a shelf of the chassis upon actuation of the inhaler and onder the shelf of the chassis upon indexing of the inhaling device. The body of the cantilever blade comprises a longitudinal axis extending from the proximal portion to the dis-tal portion of the cantilever blade, and the flexible portion extends transverse to the longitudinal axis following a transverse axis. The cantilever blade also comprises a first surface and a second surface extending along the longitudinal axis of the cantilever blade, the first surface opposite the second surface such that a first gap is formed between the priming member and the first surface of the cantilever blade and a second gap is formed between the second surface and the priming member. The distal end of the cantilever blade comprises an angled tip, which, in some aspects, can be chamfered. In some aspects, the cantilever blade has a wedge shape or a rectangular shape.

This application also provides an inhaler for inhaling a dose of powder. The inhaler includes a mouthpiece for inhaling the dose of powder and a priming member including a cantilever blade configured to engage a chassis of the inhaler. The cantilever blade of the priming member has a proximal portion, a body and a distal portion, the body heilig disposed between the proximal portion and the distal portion of the cantilever blade and the cantilever Wade having a fiexible portion extending with the proximal portion of the cantilever blade to allow movement of the cantilever blade relative to the priming member fluidly coupled to the mouthpiece.

A chassis for an inhaler is also provided. The chassis comprises a plate having blade members disposed adjacent a first aperture, a pivot shaft configured for supporting a priming member, a peripheral casing configured for rotatably supporting a pocket having a medicament; a clip having a second aperture for receiving a wheel rotatably mounted on the chassis on an axis offset from a central axis, and a shelf disposed adjacent the second aperture, the shelf defining a third aperture, the third aperture configured for receiving a cantilever blade of the priming member of the inhaler. The chassis further comprises a plurality of radially located ribs on the chassis and a plurality of peripherally located buttresses on the chassis configured for stabilizing the chassis and the priming member of the inhaler. In some embodiments, the third aperture is enclosed by a wall at one end and by an edge support at the other end. In other embodiments, the chassis further comprises a lower step and a higher step, the steps peripherally positioned on the chassis to allow molding.

In various embodiments, an inhaler is provided, the inhaler comprising a chassis having abutment members and a hollow pivot, a dispensing mechanism and an indexing mechanism, the chassis supporting the dispensing mechanism and the indexing mechanism, the dispensing mechanism comprising a priming member which comprises a priming lever, a first prodger and a second prodger mounted on the priming member, a central cam moveable as part of the indexing mechanism, a pivot opening adapted to be rotatably supported by the hollow pivot of the chassis, a driving member and a rib, the rib joining the central cam to the driving member; and the indexing mechanism comprises a Geneva wheel rotatably mounted on the chassis on an axis offset from a central axis. In some applications, the driving member further comprises a rigid cantilever blade affixed to the priming member by a hinge flexure, the cantilever blade adapted to pass over the shelf of the chassis upon actuation of the inhaling device and under the shelf upon indexing of the inhaling device. In other applications, the first prodger and second prodger are moveable towards and away from a second side surface of a supported first and second carriers of an inhaling device between a retracted and an extended position.

In various embodiments, the priming member further comprises an elongated cam member extending from the central cam towards a recess disposed opposite the leading portion of the driving member of the priming member, a lateral cam surface and at least an elongated opening, the at least an elongate opening of the priming member and the shelf of the chassis being arranged so to hold the prodgers rotationally to allow them to move towards and away from the supported first and second carriers by means of the central cam and the lateral cam surface. The Geneva wheel includes a peg wheel and two gears coaxial with the peg wheel, the peg wheel adapted to cooperate with the priming member. In many aspects, the indexing mechanism is arranged for moving a first support and a second support relative to the first and second prodgers so as to selectively align the pockets of the carriers with the respective prodgers.

In various aspects, when the priming lever begins an actuation stroke, if the priming lever is not pushed to the end of the stroke, the priming lever will return on the top of the shelf without indexing the dose carrier or the counter and the counter temporarily shows one index behind the number of doses taken. In other aspects, when the priming lever drops off the end of the shelf of the chassis at the end of an actuation stroke, the priming lever passes under the shelf of the chassis to engage the Geneva wheel advancing the dose carrier and decrementing the counter and the counter correctly represents the number of doses taken.

In some embodiments, a method of treating a respiratory disease is provided. The method of treatment comprises inhaling a powder for treating the respiratory disease from an inhaler, the inhaler comprising a mouthpiece for inhaling the dose of powder and a priming member comprising a cantilever blade configured to engage a chassis of the inhaler, the priming member having a proximal portion, a tapered body and a distal portion, the tapered body being disposed between the proximal portion and the distal portion of the cantilever blade and the cantilever blade having a flexible portion extending with the proximal portion of the cantilever blade to allow movement of the cantilever blade relative to the priming member fluidly coupled to the mouthpiece.

In other aspects, a method of treating a respiratory disease is provided. This method of treatment comprises inhaling a powder for treating the respiratory disease from an inhaler, the inhaler comprising a chassis having abutment members and a hollow pivot, a dispensing mechanism and an indexing mechanism, the chassis supporting the dispensing mechanism and the indexing mechanism, the dispensing mechanism comprising a priming member which comprises a priming lever, a first prodger and a second prodger mounted on the priming member, a central cam moveable as part of the indexing mechanism, a pivot opening adapted to be rotatably supported by the hollow pivot of the chassis, a driving member and a rib, the rib joining the central cam to the driving member; and the indexing mechanism comprises a Geneva wheel rotatably mounted on the chassis on an axis offset from a central axis. The driving member utilized in this method further comprises a rigid cantilever blade affixed to the priming member by a hinge flexure, the cantilever blade adapted to pass over the shelf of the chassis upon actuation of the inhaling device and under the shelf upon indexing of the inhaling device.

In other embodiments, a method of loading an inhaler is provided. The method of loading the inhaler includes inserting a carrier into the inhaler, the carrier comprising a plurality of pockets, each of the plurality of pockets containing individual doses of powder for inhalation, the inhaler comprising a mouthpiece for inhaling the dose of powder; and a priming member comprising a cantilever blade configured to engage a chassis of the inhaler, the priming member having a proximal portion, a tapered body and a distal portion, the tapered body being disposed between the proximal portion and the distal portion of the cantilever blade and the cantilever blade having a flexible portion extending with the proximal portion of the cantilever blade to allow movement of the cantilever blade relative to the priming member fluidly coupled to the mouthpiece. In some aspects, the method of loading an inhaler includes inserting a carrier into the inhaler, the carrier comprising a plurality of pockets, each of the plurality of pockets containing individual doses of powder for inhalation, the inhaler comprising a chassis having abutment members and a hollow pivot, a dispensing mechanism and an indexing mechanism, the chassis comprising a shelf and supporting the dispensing mechanism and the indexing mechanism; the dispensing mechanism comprising a priming member which comprises a priming lever, a first prodger and a second prodger mounted on the priming member, a central cam moveable as part of the indexing mechanism, a pivot opening adapted to be rotatably supported by the hollow pivot of the chassis, a driving member and a rib, the rib joining the central cam to the driving member; and the indexing mechanism comprises a Geneva wheel rotatably mounted on the chassis on an axis offset from a central axis. In other aspects, the driving member of the priming member further comprising a rigid cantilever blade affixed to the priming member by a hinge flexure, the cantilever blade adapted to pass over the shelf of the chassis upon actuation of the inhaling device and under the shelf upon indexing of the inhaling device.

Other features and advantages of the present disclosure will become apparent from the following detailed description. It should be understood, however, that the detailed description and the specific examples, while indicating several embodiments of the disclosure, are given by way of illustration only, since various changes and modifications within the scope of the disclosure will become apparent to those skilled in the art from this detailed description.

### BRIEF DESCRIPTION OF THE FIGURES

In part, other aspects, features, benefits and advantages of the embodiments will be apparent with regard to the following description, appended claims, and accompanying drawings.
FIGS. 1(a) to 1(c) illustrate operation of an embodiment of an assembled inhaling device or inhaler according to this application;
FIGS. 2(a) and 2(b) illustrate a carrier to load in an inhaler for use with this application without and with its lidding sheets;
FIGS. 3(a) and (b) illustrate movement of an insert from the carrier of FIGS. 2(a) to (b);
FIGS. 4(a) and (b) illustrate an arrangement for carriers within the device without and with supports of the device;
FIGS. 5(a) and (b) illustrate airway plates and anvil plates of the device in conjunction with corresponding carriers;
FIG. 6 illustrates an insert of a carrier pushed into its corresponding anvil plate;
FIG. 7(a) and (b) illustrate movement of an insert of a carrier plate into a corresponding anvil plate;
FIGS. 8(a) and 8(b) illustrates airflow paths through the embodiment of FIG. 9;
FIG. 9 illustrates the chassis and priming member assembly of one embodiment;
FIG. 10 illustrates a graph showing the force profile and operation of the embodiment of FIG. 9;
FIG. 11 is an exploded view in perspective of an inhaler illustrated in FIGS. 1(a) to 1(c);
FIG. 12 illustrates another embodiment of a chassis of the actuating mechanism of an inhaler;
FIG. 13 illustrates another embodiment of a chassis of the actuating mechanism of an inhaler;
FIG. 14 illustrates another embodiment of the priming member of the actuating mechanism of an inhaler;
FIG. 15 illustrates another embodiment of the priming member of the actuating mechanism of an inhaler;
FIG. 16 illustrates one embodiment of a cantilever blade of the priming member;
FIG. 17 illustrates another embodiment of a cantilever blade of the priming member; and
FIG. 18 is a graph showing the force profile and operation of an embodiment of an actuating mechanism illustrated FIGS. 12-17.
FIG. 19 illustrates an embodiment of a chassis, which is disk-shaped coupled to the priming member, where the priming member has an angled cantilever having a gap.
FIG. 20 illustrates an embodiment of a chassis, which is disk-shaped coupled to the priming member, where the priming member has an angled triangular cantilever having two gaps.
FIG. 21 illustrates an embodiment of a chassis, which is disk-shaped coupled to the priming member, where the priming member has an angled triangular cantilever having two gaps.
FIG. 22 illustrates the priming member of FIG. 15 with the cantilever blade embodiment of FIG. 17. The priming member is coupled to an embodiment of a chassis, which is disk-shaped.
FIG. 23 illustrates an enlarged view of an angled chamfered leading edge of the chassis shelf of the chassis of FIG. 22.
FIG. 24 illustrates an enlarged view of the tip of the cantilever blade of FIG. 22 engaged with the chassis shelf of FIG. 23.
FIG. 25 illustrates movement of the cantilever blade tip as it engages the trailing edge of the chassis shelf.
FIG. 26 illustrates movement of the cantilever blade tip as it engages the trailing edge of the chassis shelf so that the blade can move under the chassis shelf.
FIG. 27 illustrates movement of the cantilever blade tip as it moves under the chassis shelf.
FIG. 28 illustrates movement of the cantilever blade tip as it moves out from under the chassis shelf and the angled chamfered leading edge of the chassis shelf.

Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

The present disclosure may be understood more readily by reference to the following detailed description of the disclosure presented in connection with the accompanying drawings, which together form a part of this disclosure. It is to be understood that this disclosure is not limited to the specific devices, methods, conditions or parameters described and/or shown herein, and that the terminology used herein is for the purpose of describing particular embodiments by way of example only and is not intended to be limiting of the disclosure. The following description is presented to enable any person skilled in the art to make and use the present disclosure.

Notwithstanding that the numerical ranges and parameters setting forth the broad scope of the application are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

### DEFINITIONS

As used in the specification and including the appended claims, the singular forms "a," "an," and "the" include the plural, and reference to a particular numerical value includes at least that particular value, unless the context clearly dictates otherwise.

Ranges may be expressed herein as from "about" or "approximately" one particular value and/or to "about" or "approximately" another particular value. When such a range is expressed, another embodiment includes from the one particular value and/or to the other particular value.

Spatially relative terms such as "under", "below", "lower", "over", "upper", and the like, are used for ease of description to explain the positioning of one element relative to a second element. These terms are intended to encompass different orientations of the device in addition to different orientations than those depicted in the figures. Further, terms such as "first", "second", or the like, are also used to describe various elements, regions, sections, etc. and are also not intended to be limiting. Like terms refer to like elements throughout the description.

As used herein, the terms "having", "containing", "including", "comprising" and the like are open ended terms that indicate the presence of stated elements or features, but do not preclude additional elements or features.

The term "medicament" includes a substance suitable for oral or nasal inhalation. The medicament can include an active pharmaceutical ingredient and an excipient.

The term "dry powder inhaler" (DPI), refers to a device that delivers medication to the lungs in the form of a dry powder. DPIs are commonly used to treat respiratory diseases such as asthma, bronchitis, emphysema and COPD. DPIs can be divided into three basic types: i) bulk powder dispensers which hold enough bulk powder for a plurality of doses; ii) single dose dispensers, for the administration of a single dose of the active compound; and iii) multiple-dose inhalers pre-loaded with quantities of active principles sufficient for longer treatment cycles.

The term "metered-dose inhaler" (MDI) refers to a device that delivers a specific amount of medication to the lungs, in the form of a short burst of aerosolized medicine that is usually self-administered by the patient via inhalation. MDIs are commonly used as delivery systems for treating asthma, chronic obstructive pulmonary disease (COPD) and other respiratory diseases.

The term "container" refers to a receptacle for a medicament. In some embodiments, the container can have one or more pockets for the medicament.

The term "unit dose", "unit dose receptacle", and/or "dose unit" refers to a container comprising a pocket(s) that contains a medicament configured to be dispensed to a patient at a particular dose.

The headings below are not meant to limit the disclosure in any way; embodiments under any one heading may be used in conjunction with embodiments under any other heading.

Reference will now be made in detail to certain embodiments of the application, examples of which are illustrated in the accompanying figures. While the application will be described in conjunction with the illustrated embodiments, it will be understood that they are not intended to limit the application to those embodiments.

### Dispensing Device

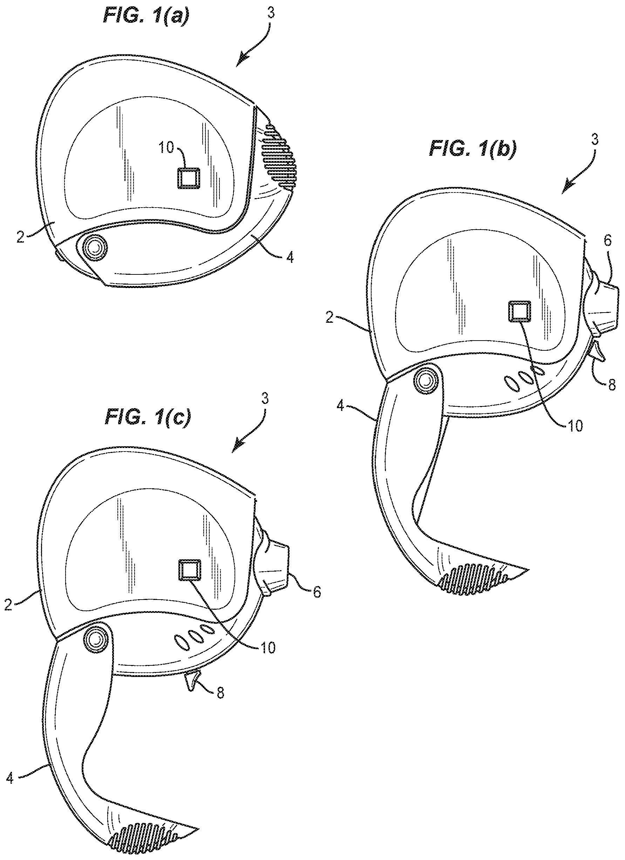

The present application provides an inhalation device from which a user may inhale consecutive doses of medicament in the form of dry powder. An embodiment is illustrated in FIGS. 1(a) to 1(c). The device includes a housing 2 on which a mouthpiece cover 4 is rotatably supported.

In order to use the device, mouthpiece cover 4 is rotated away from housing 2. As illustrated in FIG. 1(b) this exposes a mouthpiece 6. Mouthpiece 6 may be formed integrally with the housing 2, but, as will be described below, it can also be formed as a separate component for mounting with the housing 2. This allows the material properties, for instance, color, of mouthpiece 6 and housing 2 to be varied easily according to the requirements of device 3.

As illustrated in FIG. 1(b), a priming lever 8 extends out of the housing 2 at a position adjacent the mouthpiece 6. Priming lever 8 is mounted so as to rotate about a central axis within the device. In this way, it is moveable by the user around a periphery of the housing 2 to a position as illustrated in FIG. 1(c). Movement of priming lever 8 from the first position illustrated in FIG. 1(b) to the second position illustrated in FIG. 1(c) is arranged to prime the device, in particular, to expose a dose of powder such that it may be carried with an airstream out of the mouthpiece 6.

It should be noted that locating the first position of priming lever 8 adjacent mouthpiece 6 is highly advantageous, since it discourages a user from attempting to inhale from mouthpiece 6 before moving priming lever 8 away from mouthpiece 6 to the second position of FIG. 1(c). In other words, the user is encouraged to prime the device before attempting to inhale through it. Nevertheless, it should be noted that a small space is provided between mouthpiece 6 and priming lever 8 so as to allow the user to operate priming lever 8 with his or her finger without touching mouthpiece 6.

After use of the device, mouthpiece cover 4 may be rotated back to its stowed position illustrated in FIG. 1(a). In this respect, an inner surface of mouthpiece cover 4 is provided with a return actuator for engaging with priming lever 8. In particular, when mouthpiece cover 4 is moved from its open position of FIGS. 1(b) and 1(c) to its closed position of FIG. 1(a), the return actuator engages with priming lever 8 and moves it back from its second position illustrated in FIG. 1(c) to its first position illustrated in FIG. 1(b). As will be described further below, in one embodiment, this movement of priming lever 8 operates an indexing mechanism for moving a still unused and unopened pocket of powder into line with a dispensing mechanism such that, with subsequent priming of the device, the powder of that pocket is dispensed for inhalation. By operating the indexing mechanism during the return movement of priming lever 8 immediately after priming and release of a pocket of powder, if the released powder is not inhaled, it is indexed to a position where it can safely be held within the device.

As illustrated in FIGS. 1(a) to 1(c), the embodiment also includes a window 10 in one side of housing 2. Window 10 is provided so as to allow a user to view a counter display within the device. A counter mechanism indexes the counter display upon each use of the device so as to provide the user with an indication of how many doses have been dispensed and/or how many doses remain unused.

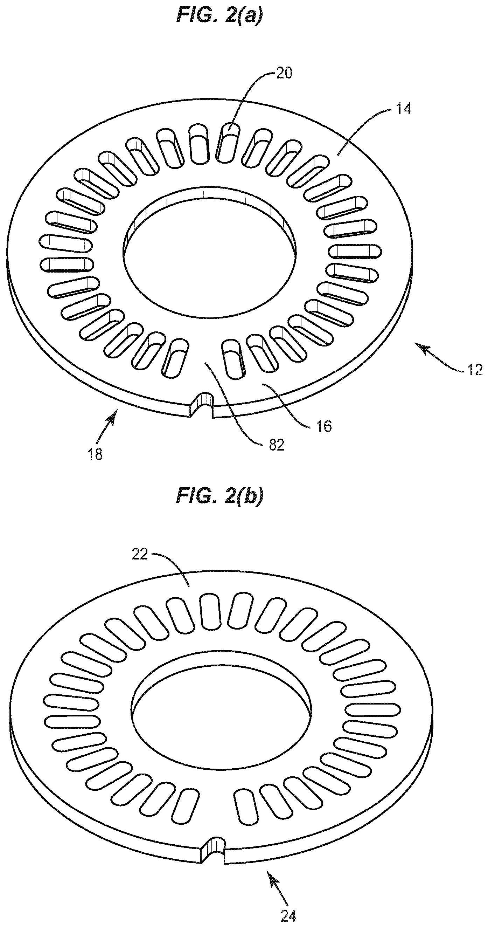

Many aspects of the present application are applicable to devices housing a wide variety of different dose carriers. In particular, many of the features of the embodiment described below can be used with carriers having a traditional blister-pack construction, with carriers having various arrays of pockets and, in some arrangements, with some carriers having a single respective pocket. Nevertheless, the present application is useful when used with carriers of the form illustrated in FIGS. 2(a) and 2(b).

As illustrated in FIG. 2(a), each carrier 12 is formed from a disk-shaped base 14 having a substantially planar first side surface 16 opposite and parallel with a substantially planar second side surface 18. A plurality of through holes 20 are formed between the first and second side surfaces 16, 18 so as to form spaces for housing doses of powder. Base 14 is formed with an appreciable thickness so as to provide the through holes 20 with sufficient space to house the required doses of powder. Through holes 20 are arranged as a circumferential array and, in one embodiment, 30 through holes are provided in the array.

As illustrated in FIG. 2(b), the first and second side surfaces 16, 18 of base 14 are sealed with respective first and second lidding sheets 22, 24. In this way, carrier 12 provides a plurality of pockets housing individual respective doses of powder.

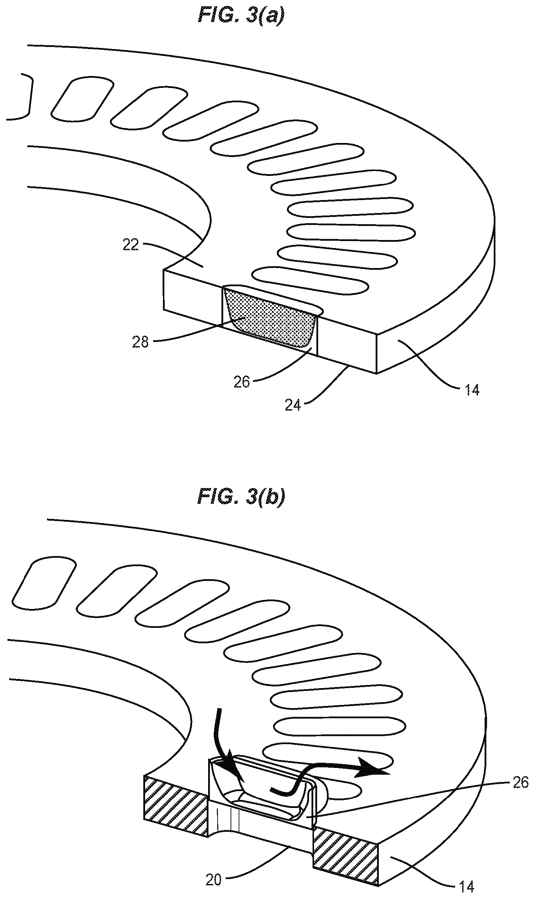

As illustrated by the cross-sections of FIGS. 3(a) and (b), the pockets include a respective insert 26 within each through hole 20. Inserts 26 are generally cup-shaped with their open ends facing first lidding sheet 22. Each insert contains a respective dose of powder 28.

By pushing on the closed end of insert 26 from the side of second lidding sheet 24, it is possible to push insert outwardly from base 14 of carrier 12 through first lidding sheet 22. This is illustrated in FIG. 3(b), but, for clarity, without either lidding sheet. As illustrated, with insert 26 extending out of base 14, it may be more convenient to provide an airflow (such as indicated by arrows) to remove powder from the pocket.

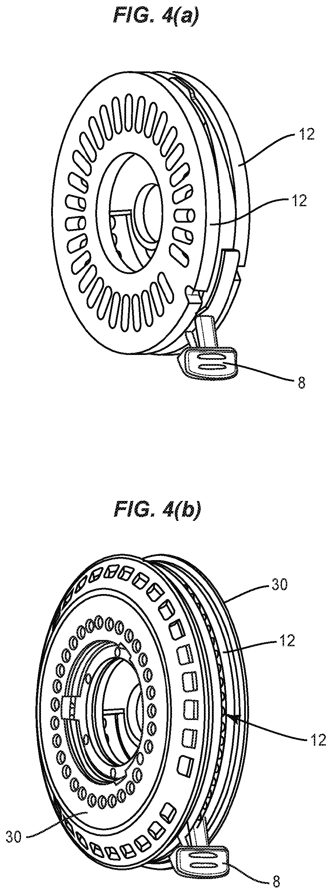

Within housing 2 of the inhalation device, in one embodiment, two of the carriers 12 are arranged coaxially side by side as illustrated in FIG. 4(a). Each carrier 12 is provided with a support 30 as illustrated in FIG. 4(b). In the illustrated embodiment, each support 30 is positioned adjacent an outwardly facing surface of its respective carrier 12. In particular, the first side surface 16 of each carrier 12 faces a respective support 30 such that a dispensing mechanism may be provided between the two carriers 12 so as to press respective pocket inserts 26 (FIGS. 3(a) and 3(b)) outwardly towards the respective supports 30. An arrangement for this embodiment will be described further below.

As illustrated, priming lever 8 is positioned such that it extends between carriers 12 and is rotatable about the common axis of carriers 12 so as to operate a dispensing mechanism and an indexing mechanism.

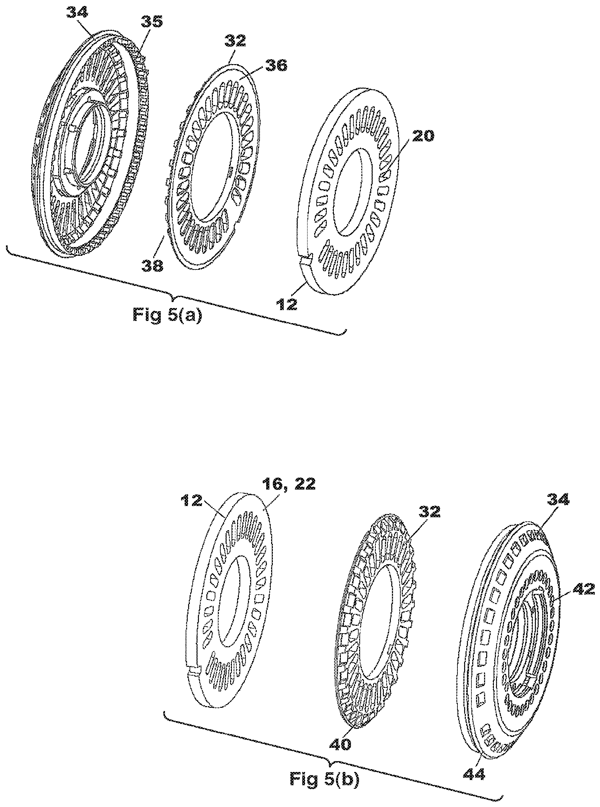

In an embodiment, each support 30 is made up of two components, namely an anvil plate 32 and an airway plate 34. These are illustrated in FIGS. 5(a) and (b) in conjunction with associated carriers 12.

Each anvil plate 32 has a planar surface 36 which, in use, abuts against the first side surface 16 of associated carrier 12 as covered by first lidding sheet 22. Each anvil plate 32 also includes a plurality of guide through holes 38 corresponding to through holes 20 of associated carrier 12.

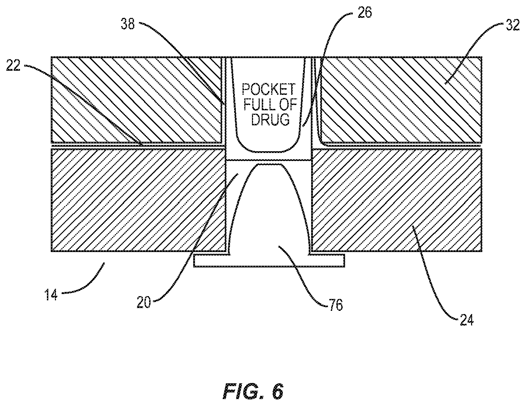

In this way, as illustrated schematically in FIG. 6, an insert 26 can be pushed out of its through hole 20 and into a corresponding guide through hole 38 of anvil plate 32. Insert 26 is thus used to outwardly burst through first lidding sheet 22, but is still held securely in place. Although not of a particular concern here, anvil plate 32 also supports first lidding sheet 22 around through hole 20 and can be used to improve the predictability of the nature of the lidding sheet rupture.

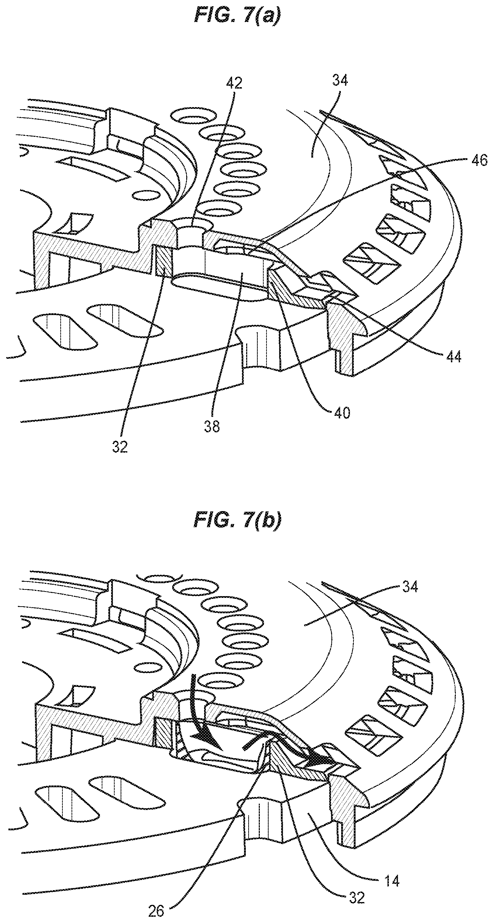

As illustrated by the cross-section of FIG. 7(a), anvil plate 32 includes a second surface 40 which abuts an inner surface of associated airway plate 34. Airway plate 34. includes a pair of through holes corresponding to each guide through hole 38 of corresponding anvil plate 32. In particular, each pair includes an inlet hole 42 and an outlet hole 44.

As illustrated in FIG. 7(a), relative to surface 40 of anvil plate 32 abutting the inner surface of airway plate 34, a recessed channel 46 extends radially inwardly from outlet 44 so as to communicate with guide through hole 38 of anvil plate 32. Hence, for each guide through hole 38 of anvil plate 32, airway plate 34 provides, communicating with it, a corresponding inlet 42 and outlet 44 with its associated recessed channel 46. In particular, each inlet 42 communicates with one side of its associated guide through hole 38 while corresponding outlet 44 communicates with the opposite side of associated guide through hole 38.

As illustrated in FIG. 7(b), when an insert 26 is pushed outwardly of through hole 20 (FIG. 6) of base 14 into guide through hole 38 of anvil plate 32, it is positioned with the open portion of its cup-shape facing inlet 42 (at one end of the cup-shape) and recessed channel 46 (at the opposite end of the cup-shape). In this way, as illustrated, an airflow may be drawn through airway plate 34 such that it passes down into the pocket formed in insert 26, back up into recessed channel 46 and then out of outlet 44. Powder in insert 26 is thus picked up by the airstream, removed from insert 26 and carried out of airway plate 34. A flow path is thus formed into and out of a pocket which may then connect the pocket to mouthpiece 6 of the device.

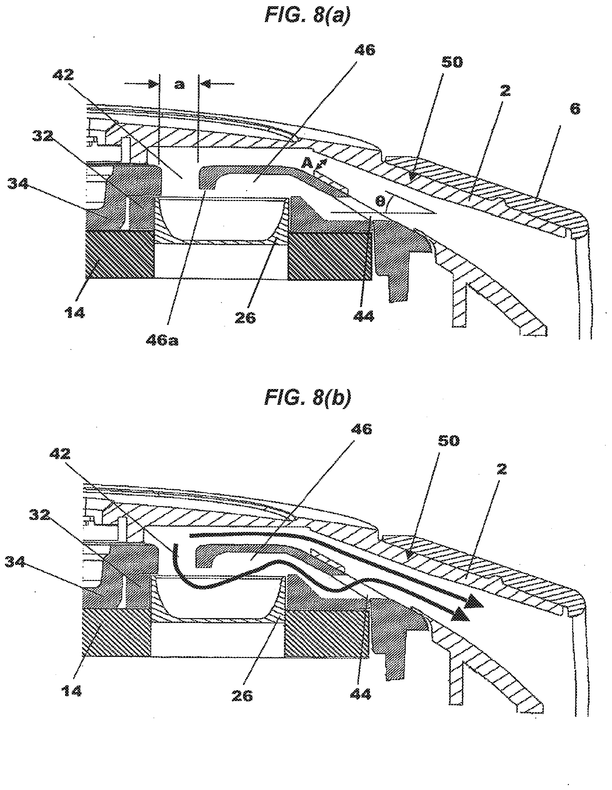

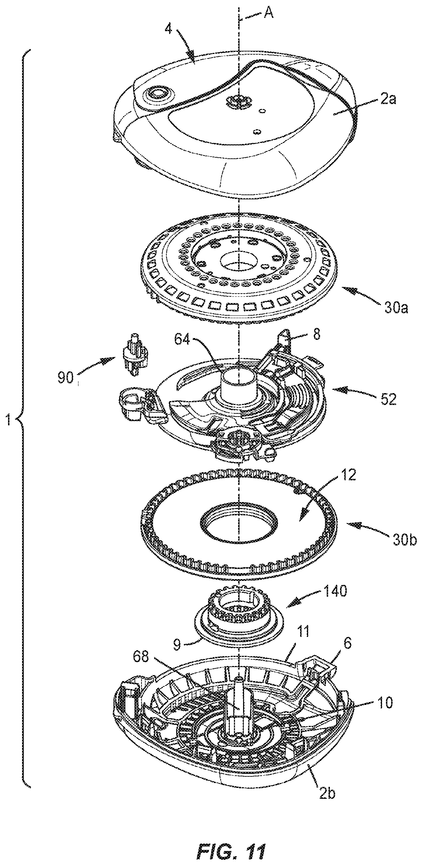

As illustrated in FIG. 11, housing 2 may be formed from a pair of casing halves 2a and 2b. As illustrated in FIGS. 8(a) and 8(b), an inner wall 50 of casing halves 2a and 2b cooperates with airway plate 34 so as to form a second flow path to mouthpiece 6 which bypasses the pocket(s). Alternatively, an additional component may be provided, to define the second flow path.

As illustrated in FIG. 8(b), for each pocket formed by an insert 26, a corresponding inlet 42 of the airway plate 34 is positioned adjacent a periphery of the pocket. Corresponding outlet 44 is provided on an opposite side of the pocket such that the airstream between inlet 42 and outlet 44 crosses the pocket and, hence, picks up any powder from the pocket. As illustrated, inlet 42 is formed as a portion which is directed down into insert 26 forming the pocket.

In this way, when a user inhales through the device and creates an airstream through it, the airstream drawn through inlet 42 will be directed down into any powder in insert 26 so as to dislodge it and move it into the airstream so as to be carried out of outlet 44. In the illustrated embodiment, recessed channel 46, which connects the volume of the pocket to outlet 44, is positioned adjacent inlet 42. In this way, the airstream from inlet 42 is deflected from the base of insert 26 (and any powder there) so as to travel back towards recessed channel 46. Powder carried in the airstream up into recessed channel 46 is subjected to a relatively sharp change in direction. As a result of this, powder in the airstream tends to be disaggregated. Furthermore, the powder will tend to hit the surfaces of recessed channel 46 also contributing to disaggregation.

As is clear from FIG. 8(b), the shape of the airway path is chosen to force large aggregates of powder to impact the walls as the airflow is forced to change direction, thereby disaggregating large clumps of powder. The shape is also designed to ensure that airflow over any surface within the airway is maintained at a high value to avoid excessive powder adhering to the surface. Thus corners are rounded and the cross section at each position along the tube is designed to maintain air velocity without generating excessive pressure drop.

As illustrated in FIG. 8(a), in this embodiment, the airflow through the pocket has its minimum area at the inlet to the pocket defined by the dimension "A" whereas the airflow that bypasses the pocket has its minimum cross section just before the airflow join and so is defined by the dimension A.

The air velocity is highest where the cross sectional area is smallest so this arrangement provides high velocity air to extract the powder from the pocket and uses the high velocity of the bypass air joining the powder contained in the pocket airflow to assist disaggregation and to protect the walls from powder deposition.

The airflow velocity through the pocket is controlled mainly by the suction pressure created as the user inhales, whereas the volume flow rate is a factor of both velocity and area.

A sufficiently high air velocity should be generated to ensure that the powder is entrained in the airflow. However, if the velocity and flow volume are too high, then there is the possibility that the whole of the mass of powder in the pocket is pushed through the airway as an agglomerated clump. If this happens, the clump may not accelerate to a sufficient velocity for its impact with the walls in the airway to break it up and provide disaggregation. It is believed that the powder is removed gradually from the pocket by the airflow. To achieve this, a small gap 46a is provided between the surface of the powder in the pocket and the airway roof formed from the division in the airway plate 34 between the inlet 42 and recessed channel 46. This, combined with a dimension for "A" that limits the flow volume through the pocket, ensures that the powder is eroded from the pocket rather than pushed out.

To enable this, the inlet hole diameter "a" is chosen to be between 0.5 mm and 2.0 mm for pockets of around 2.0 mm width (in a circumferential direction) and of around 7.3 mm length (in a radial direction). The value chosen depends on the properties of the powder.

In this way, the powder can be removed from the pocket over a time period ranging from between 0.1 s to 1.0 s. This is within the period of the high flow rate of the inhalation cycle and provides good disaggregation of the powder.

It should be appreciated that, in other embodiments, it is possible for parts of the flow path through the pocket, other than the inlet hole, for instance downstream of the powder, to form the minimum cross-sectional area of that flow path. Similar considerations will still apply for the diameter "a" of the inlet hole.

The arrangement of inlet hole 42 and channel 46 is particularly advantageous in conjunction with deep narrow pockets of powder. At a particular flow rate, for instance 10 ltr/min, the surface of the powder will be eroded by a certain depth. Increasing the flow rate to, for instance 20 ltr/min, will result in the powder being eroded by a further depth. Since inhalation by users results in flow rates which increase progressively to a maximum, powder is eroded depth by depth and the pocket is emptied gradually over an appropriate period.

Although the volume and strength of inhalation will vary between users, it is important that the device should not provide too much in the way of resistance to inhalation. In this respect, it would be extremely difficult to inhale through an inlet 42 having a desired cross sectional area. Indeed, where possible, it would result in a flow velocity which was far too high and which would entrain of all of the powder from insert 26 far too quickly. In practice, it is found that approximately only 20% of inhaled air can be used directly for picking up and disaggregating the powder.

As illustrated in FIG. 8(b), a second flow path is formed between an inner wall 50 of housing 2 and the outside of airway plate 34. The second flow path bypasses the pocket and increases the overall cross sectional area available through which to inhale. By changing the values of the dimensions a and A, it is possible to change the rates of airflow between the pocket and bypass and to control the overall flow resistance of the device so that it is comfortable for the user to inhale through. A typical flow resistance for the device would be between 2 kPa and 5 kPa for a flow volume of 601/min. Higher flow resistances are chosen for powders which are harder to disaggregate, whereas lower flow resistances are useful for devices used by children. Recessed channel 46 and outlet 44 generally have larger cross sectional areas than the inlet 42. It is envisaged that the minimum cross sectional area for the pocket path would be 3.5 mm² to 4.0 mm² and for the bypass 5.0 mm² to 6.0 mm².

In this way, it is relatively easy to inhale through the device, since a large proportion of the airflow will be through the second flow path. Nevertheless, some of the flow will occur through the first flow path so as to entrain and disaggregate the powder as described above.

In one embodiment, there is another second flow path for the other side of the device and its corresponding carrier. In use, a patient inhales through both second flow paths while drawing powder from the first flow path. Each of the second flow paths is expected to carry approximately 40% of the total inhaled air.

Actual requirements will vary depending upon the nature of the powder and the intended user. For an easily dispensed powder, the portion forming the inlet to the pocket can be small and, for a child or patient with COPD (Chronic Obstructive Pulmonary Disease), the total pressure drop should be low. In this case, an inlet portion could be provided with a cross-sectional area of 2 mm² and a bypass second flow path with a minimum cross-sectional area of 8 mm², resulting in a ratio of 25%). On the other hand, with sticky powder for a healthy adult, the inlet portion could be provided with a cross-sectional area of 4 mm² together with a bypass second flow path having a minimum cross-sectional area of 6 mm², resulting in a ratio of 66%. Of course, intermediate values are also possible and a useful arrangement has an inlet portion of approximately 3 mm² with a second flow path minimum cross-sectional area of 6 mm², resulting in a ratio of 50%.

As illustrated in FIG. 8(a), the walls of the outlet 44 are orientated so as to direct the flow of air and powder into the second flow path at an angle θ relative to the flow in the second flow path. By ensuring that the angle θ is less than 45°, it is possible to substantially reduce the amount of powder which might impact with or stick to the wall 50 opposite the outlet 44. In some embodiments, the angle θ is no greater than 45° and, in other embodiments, no greater than 30°. In this way, substantially no powder will adhere to the wall 50 forming the second flow path to the mouthpiece 6. In some aspects, with repeated use of the device, no more than 25%, in other aspects, no more than 15% of a dose remains deposited on the wall 50. In this respect, it will be appreciated that the flow from the bypass past wall 50 will act to scour or scavenge powder from the wall 50.

As mentioned above with reference to FIG. 5(b), the anvil plate 32 and airway plate 34 together form a support for a corresponding carrier 12. By means of the priming lever 8 and the indexing mechanism to be described below, a support 30 and its corresponding carrier 12 is moved to consecutive positions to dispense powder from consecutive pockets. In this regard, it will be appreciated that each pocket has its own first flow path as formed in airway plate 34. From the description above, it will be appreciated that turbulent flow in removing powder from the pocket and disaggregation of powder occurs within the first flow path. Thus, should any powder adhere to walls within airway plate 34, this powder is not available for inhalation when subsequent pockets of powder are dispensed.

In some embodiments, the device is arranged such that an inlet passage that provides the air for the flow through the pocket and through the bypass is arranged so that it feeds the air only to the pocket positioned for dispensing, such as illustrated in FIGS. 8(a) and 8(b). The indexing of carrier 12, anvil plate 32 and airway plate 34 after use repositions inlet 42 and outlet 44 for a used pocket outside of the airflow for the pocket currently in use.

This arrangement ensures that, even if none of the powder from a pocket is removed after it has been opened, once it has been indexed on, then the powder will be permanently retained within the device such that it will not be inhaled along with a subsequent dose.

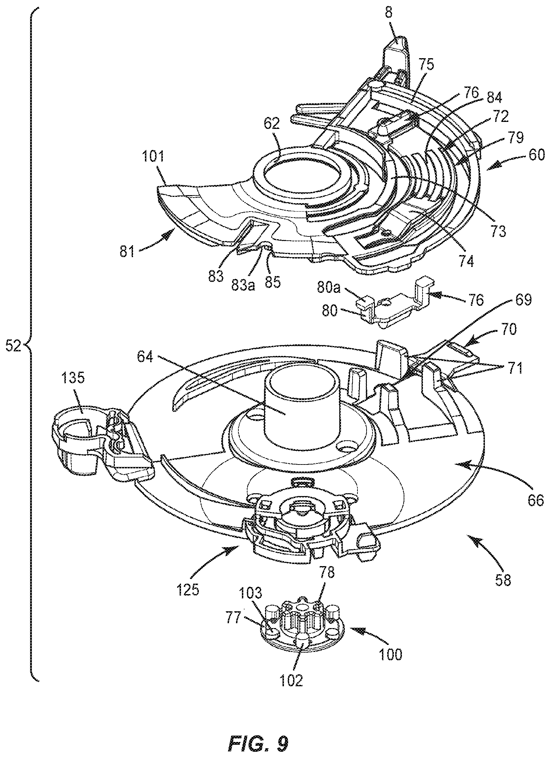

Supports 30 and associated carriers 12 may be rotatably mounted within housing 2 by means of a chassis assembly 58 as illustrated in FIG. 9. The chassis assembly 58 is positioned between second side surfaces 18 of carriers 12. It extends axially along the axis of carriers 12 and is fixed to one or both of the two halves 2a, 2b of housing 2.

As illustrated in FIG. 9, priming lever 8 forms part of (or could be attached to) a priming member 60. Priming member 60 has a central pivot opening 62 by which it is rotatably supported on a pivot shaft 64 of a chassis 66.

With reference to FIG. 9 and 11, priming member 60 and chassis 66 are together positioned as an actuating mechanism 52 between the two carriers 12 and associated supports 30a and 30b. Furthermore, chassis 66 is mounted to housing 2 so as to be rotatably fixed. In the illustrated embodiment, pivot shaft 64 may itself be located on a shaft 68 (shown in FIG. 11) provided on the inside of one or both halves 2a, 2b of the housing 2. Also, a radial extension 70 (shown in FIG. 9) may be provided on the chassis 66 to interact with an inner portion of housing 2 so as to rotationally fix chassis 66. Carriers 12 and associated supports 30a and 30b may be rotationally mounted on chassis 66.

Priming member 60 includes an elongate cam member 72 which extends in a circumferential direction and has a cam surface 74 on each of two opposite sides.

Each cam surface 74 interacts with a respective member 76 which will be described as a prodger.

As can be seen in FIG. 9, priming member 60 includes elongate openings 79 on either side of the cam member 72 through which arms 80 of prodgers 76 can extend. The chassis 66 holds prodger 76 rotationally but allows them to move in an axial direction of the device, in other words towards and away from carriers 12 on either side. Indeed, as illustrated, an aperture 69 exists in chassis 66 allowing one of prodgers 76 to extend through chassis 66 towards a corresponding carrier 12.

Referring to FIG. 2(a), it will be seen that, in one aspect, carrier 12 has an array of through holes 20 which includes a blank portion 82 in which a through hole 20 is not formed.

Using carriers of this type, it is possible to position one carrier 12 with the blank portion 82 opposite a prodger 76 while consecutively indexing the other carrier 12 around each of its through holes 20 and the pockets they form until all have been emptied. The indexing mechanism can then rotate the empty carrier to a position in which its blank portion 82 is opposite prodger 76 and rotate the other carrier 12 around all of the positions in which corresponding prodger 76 aligns with through holes 20. In this way, the same dispensing mechanism is used for dispensing powder from both carriers and using the same operation.

Although it is the intention that substantially all of the powder dispensed from the individual pockets will be removed from the device by way of inhalation, it is possible that some powder will remain within the device. Indeed, where different types of carrier are used or the device has a different application, it might be that more powder does remain within the device.

As illustrated in FIG. 9, cam surfaces 74 are provided with one or more grooves or channels 84. Any excess powder can thus fall into the grooves or channels 84 such that contact and movement between cam surface 74 and prodger 76 is not impeded.

The actuating mechanism 52, illustrated in detail on FIG. 9, is arranged between the bottom surfaces of the carriers 12. Actuating mechanism 52 is adapted to expose one of unitary doses 28 of dry powder such that it may be carried with the airstream out of the mouthpiece 6 each time priming lever 8 is actuated.

In particular, actuating mechanism 52 comprises a dispensing mechanism adapted to expose each unitary dose 28 to the corresponding flow path, and an indexing mechanism adapted to place each flow path in communication with mouthpiece 6.

Actuating mechanism 52 comprises a disk-shaped chassis 66 which supports the dispensing mechanism and the indexing mechanism. The chassis is fixed to casing 2 and comprises a hollow pivot shaft 64 fitted on shaft 68 of casing 2. At a location, the chassis comprises guide members 71, extending axially and defining a radial aperture between them. Chassis 66 is also provided with a radial extension 70 which interacts with an inner portion of housing 2 so as to rotationally fix chassis 66.

Actuating mechanism 52 further comprises a priming member 60 bearing the priming lever 8 and rotatable about the central axis A so as to operate the dispensing mechanism and the indexing mechanism when the priming lever 8 is actuated.

An example of a suitable priming member 60 is disclosed in WO-A-2005/002654. Priming member 60 is formed of a disk-shaped plate molded in plastic and having a central pivot opening 62 by which it is rotatably supported on pivot shaft 64 of chassis 66.

In the illustrated embodiment, the dispensing mechanism is adapted to move each pocket insert 26 of each carrier 12 from its storage position to its discharge position. Again, an example of a suitable dispensing mechanism, implementing prodgers 76 mounted on the priming member 60, and cam surfaces 74, 73 arranged on priming member 60 and adapted to move prodgers 76 axially, is disclosed in WO-A-2005/002654.

In particular, the dispensing mechanism includes an elongate cam member 72 formed on priming member 60 and separated from the remaining part of priming member 60 by grooves or channels 84 through which guide members 71 of chassis 66 extend. Cam member 72 extends in a circumferential direction and presents a profile adapted to provide a limited amount of flexibility. The central cam surface 74 is provided on each of two opposite sides of the cam member 72. Besides, lateral cam surfaces 73, 75 extend on either side of the priming member 60, in circumferential directions along the elongate openings 79, opposite cam member 72.

Prodgers 76 are identical to each other and clip together with cam member 72 between them. Each prodger 76 has arms 80 extending perpendicularly to a central part arranged to cooperate with central cam surface 74 of cam member 72. Arms 80 extend through elongate openings 79 of priming member 60, and have features 80a arranged at their ends to contact lateral cam surfaces 73 of priming member 60.

Elongate cam openings 79 and grooves or channels 84 of the priming member 60, and guide members 71 on the chassis 66 are arranged to hold prodgers 76 rotationally, but to allow them to move in an axial direction of the device 3, towards and away from carriers 12 by means of central 74 and lateral 73 cam surfaces that positively guide prodgers 76.

As explained in WO-A-2005/002654, actuating mechanism 52 arranges for one of prodger 76 to be in alignment with one of insert 26 of corresponding carrier 12 while other prodger 76 faces blank portion 82 of other carrier 12. In this way, the dispensing mechanism only dispenses one unitary dose 28 of one of carrier 12 at a time.

Operation of the dispensing mechanism is now described.

As illustrated in FIG. 11, movement of priming lever 8 in slot 11 of casing 2b along its stroke from a first position close to mouthpiece 6 to a second position at a distance from mouthpiece 6 primes inhaler 1 to expose unitary dose 28 of dry powder to the corresponding flow path.

At an initial step, when the user moves mouthpiece cover 4 to expose mouthpiece 6, priming lever 8 is in its first position and both prodgers 76 are in a retracted position at one end of cam member 72 opposite central cam surfaces 74.

When the user moves priming lever 8 to its second position, priming member 60 is rotated relative to chassis 66. Cam surfaces 74 of cam member 72 engage prodgers 76, respectively. Cam surface 74 that engages prodger 76 in alignment with one of insert 26 presses out prodger 76 so that prodger 76 is moved outwardly towards its corresponding carrier 12, penetrates through-hole 20 of carrier 12 and pushes insert 26 in the discharge position. Meanwhile, cam surface 74 that engages prodger 76 in alignment with blank portion 82 deforms thanks to its flexibility.

After the user has inhaled unitary dose 28, mouthpiece cover 4 may be rotated back by the user. The actuation rib of mouthpiece cover 4 may engage priming lever 8 to move it back to its first position. Lateral cam surfaces 73 of priming member 60 retract prodgers 76.

The indexing mechanism will now be described.

In the embodiment illustrated in FIG. 11, the indexing mechanism is adapted to move the first 30a and second 30b supports in successive active positions in each of which one of the flow paths is connected to mouthpiece 6 so that corresponding unitary dose 28 may be carried by the airstream through mouthpiece 6. An example of a suitable indexing mechanism implementing an intermittent motion mechanism is disclosed in WO-A-2005/002654.

In particular, as illustrated in FIG. 9, the indexing mechanism comprises a Geneva wheel 100 rotatably mounted within casing 2 about an axis parallel to the central axis A. Geneva wheel 100 includes a peg wheel 77 adapted to cooperate with priming member 60 so that the Geneva wheel rotates through an angle of 120° each time priming lever 8 is actuated. Geneva wheel 100 also includes two gears 78 coaxial with peg wheel 77 and adapted to cooperate respectively with the coupling portions of first 30a and second 30b supports. Peg wheel 77 has three long pegs 102 and three short pegs 103 arranged alternately at intervals of 60° around its edge.

The indexing mechanism further comprises a driving member 81 formed on an outer edge of priming member 60. Driving member 81 is arranged so that when priming lever 8 is moved from its first position to its second position as explained above, the dispensing mechanism push pocket insert 26 (not shown) in the discharge position, driving member 81 does not rotate Geneva wheel 100, when priming lever 8 is moved back from its second position to its first position, driving member 81 rotates Geneva wheel 100. In particular, driving member 81 is placed, in the circumferential direction, next to a portion of priming member 60 comprising the dispensing mechanism.

Driving member 81 is provided with a leading portion 101, a ratchet pawl 83 which slopes downward toward leading portion 101, and a slot 83a with a trailing edge 85 arranged in sequence. The operation of the indexing mechanism will now be described in relation to one cycle defined by the movement of priming lever 8 as it is actuated by the user. The terms "first", "second" and "third" related to long 102 and short 103 pegs in the following description are used in relation to one cycle. It should be understood that the "first", "second" and "third" pegs would change in a subsequent cycle.

As indicated above, when priming lever 8 is moved from its first position to its second position, driving member 81 does not rotate Geneva wheel 100. In particular, peg wheel 77 and driving member 81 are arranged so that the outer edge of priming member 60 passes over the first of short pegs 103 and slides against the first and second of long pegs 102 adjacent on either side of first short peg 103, ratchet pawl 83 deforming when passing over second short peg 103. Geneva wheel 100 is therefore prevented from rotating.

When priming lever 8 returns from its second position to its first position, leading portion 101 passes over first short peg 103 and the outer edge of priming member 60 slides against first and second long pegs 102, thereby preventing peg wheel 77 from rotating. Then ratchet pawl 83 engages with first short peg 103 so that peg wheel 77 is driven around, second long peg 102 entering slot 83a. As ratchet pawl 83 disengages first short peg 103, trailing edge 85 of slot 83a engages second long peg 102 and continues to drive peg wheel 77 around. As trailing edge 85 of slot 83a disengages the second long peg 102, the outer edge of priming member 60 passes over second of short pegs 103 adjacent to second long peg 102 and abuts against second and third of long pegs 102. The indexing mechanism causes one of each carrier 12 to be incremented by one unitary dose 28 each time priming lever 4 is actuated.

Gear teeth 35 of the coupling portion of each airway plate 34. may be in engagement with corresponding gear 78 of Geneva wheel 100 so as to be moved with respect to casing 2 successively in the active positions. The numbers of gear teeth 35 on airway plates 34. and gears 78 are arranged so that the motion of an angle of 120° of Geneva wheel 100 increments support 30a or 30b exactly one pitch.

To avoid having both first 30a and second 30b supports driven simultaneously, the indexing mechanism is caused initially to drive first support 30a and, when this has had all of its unitary doses 28 dispensed, to then drive second support 30b.

The embodiment described above is arranged to dispense the dry powder from each insert 26 of one carrier 12 and then subsequently the dry powder from each insert 26 of other carrier 12. However, it should be appreciated that it is also possible for a device to dispense dry powder from inserts 26 alternately from one carrier 12 and then other carrier 12. Alternatively, inserts 26 of both carriers may be dispensed simultaneously.

It will be appreciated that, with the arrangement where one or other of prodgers 76 abuts a blank portion 82 of a carrier 12 where there is no pocket, in order for priming member 60 to rotate and cam member 72 to move a prodger 76 towards other carrier 12, it will be necessary for cam member 72 to move away from blank portion 82. In some embodiments, it might be possible to allow the entire priming member 60 to move axially or for carriers 12 to move axially. However, in one embodiment, cam member 72 has itself a limited amount of flexibility. As illustrated, cam member 72 is provided as an elongate member which is attached to rest of priming member 60 at each end with an elongate opening either side of it. This will allow sufficient flexibility for cam member 72 to move towards and away from carriers 12.

Considering the overall embodiment as described with reference to FIG. 1(a) to (c), it will be appreciated that it is highly desirable to ensure that the user moves the priming lever 8 through its entire length of travel so as to fully dispense a dose of powder. In particular, it would be undesirable for a user to partly operate priming lever 8 and priming member 60 such that a prodger 76 pushes an insert 26 far enough to partly rupture a lidding sheet on first side surface 16, but without fully extending insert 26 to the position illustrated in FIGS. 6 and 7(b).

In the embodiment illustrated in FIG. 9, the indexing mechanism is initiated through engagement of priming lever 8 of priming member 60 with index Geneva short pin 103; rotation of index Geneva wheel 100 causes the carrier disk to advance to the next dose position and the counters to decrement by 1. In this application, priming lever 8 on priming member 60 in device 3 follows the same path on both actuation and indexing, and uses a ratchet action to skip over the index Geneva short pin 103 on the actuation stroke. The path followed by priming member 60 is under the chassis throughout the actuation step including indexing and counting.

As the motion of the inserts 26 is restricted by first and second lidding sheets or foils 22, 24, sealing both surfaces of carrier plate 12, a high force is required to cause inserts 26 to start to move. This force increases to the point at which foils 22, 24 rupture after which the force decreases substantially. Thus, the user feels a resistance to the motion of priming lever 8 for the early part of its travel. At some point along its travel, the resistance suddenly reduces, as foil 22, 24 rupture. The user cannot reduce the applied force instantaneously so that priming lever 8 is rapidly pushed to the end of its available stroke. This tactile feedback encourages the user to fully open the pockets.

In this way, reliable opening of the pocket is achieved using components that can be manufactured using conventional materials and molding processes.

In one embodiment, the indexing of the two carrier assemblies (FIGS. 5(a) and (b)) is accomplished by an indexing mechanism that causes a carrier 12 to be incremented by one pocket each time priming lever 8 is actuated and a changeover mechanism that causes the indexing mechanism initially to drive first carrier 12 but, when the last pocket of that carrier 12 has been used, for that carrier 12 to remain stationary while second carrier 12 is incremented when the indexing mechanism is actuated.

A useful indexing mechanism described in WO-A-2005/002654 uses a 3 peg Geneva wheel 100 that rotates exactly 120° each time the indexing mechanism is actuated. The Geneva wheel 100 has two gears co-axial with the peg-wheel arranged so that the gears can engage with teeth 35 on the airway plates 34.

To avoid having both airway plates 34 driven simultaneously, it is arranged that, at one location around airway plate 34, gear teeth 35 are missing. As a result, at this location, rotation of Geneva wheel 100 does not rotate airway plate 34. Thus, the indexing mechanism drives first carrier 12 via Geneva wheel 100 and its gears until it reaches the end of gear teeth 35 for that carrier 12. The next indexing moves first carrier 12 to its non-driven position, i.e. where gear teeth 35 are missing, and engages a changeover mechanism which rotates second carrier 12 until its gears 35 are engaged with the gears on Geneva wheel 100.

Rotary priming member 60 incorporates many of the functional elements described previously within a single molded component. It includes priming lever 8, cam member 74, prodger 76, and ratchet 83, as well as being the driving member for indexing Geneva wheel 100.

As described previously, for the device to operate with two disk carrier plates, in some applications, a changeover mechanism is provided to cause the indexing mechanism initially to drive a first disk and, when this has had all of its pockets opened, to then drive a second disk. Such a changeover mechanism is described in WO-A-2005/002654. The changeover mechanism allows the same indexing mechanism to initially index a first carrier disk and then, at a predetermined location, index both carrier disks together for one increment and then subsequently cause the indexing mechanism to only index the second carrier disk. The changeover action can be initiated solely by the angular position of the first carrier disk requiring no other input from the user and providing insignificant difference in the tactile feedback. In one embodiment, the indexing of the two carrier assemblies (FIGS. 5( a) and (b)) is accomplished by an indexing mechanism that causes a carrier 12 to be incremented by one pocket each time priming lever 8 is actuated and a changeover mechanism 90, illustrated in FIG. 11 that causes the indexing mechanism initially to drive first carrier 12 but, when the last pocket of that carrier 12 has been used, for that carrier 12 to remain stationary while second carrier 12 is incremented when the indexing mechanism is actuated. For example, a casing 135, visible on FIG. 9, may be formed in one piece with chassis 66 of actuating mechanism 52 to rotatably support a component of the changeover mechanism.

Clip 125 also shown in FIG. 9 provides an interlock that prevents any frictional coupling from causing the upper airway plate of a two disk device to move before the lower airway plate has arrived at the correct location.

Thus, changeover from the indexing of one disk to the other is achieved automatically and with minimal number of components and in a very small space.

The indexing of the device, in addition to moving the next pocket into alignment with prodgers 76, in some aspects, it actuates a dose counter that provides a visual indication to the user of the number of doses remaining. The operation of a useful dose indicator for the embodiment illustrated in FIG. 9 is described in WO-A-2005/002654.

It is useful that the device, when dispensing medicament, indicates to the user the number of doses remaining in the device. It is also useful that such indication is easily readable and, as such, very small numbers indicating the remaining doses would be a disadvantage. Within the size constraints of a pocket portable device that contains 60 doses providing such a display is challenging.

The simplest arrangement of marking the carrier disks with numbers visible through windows in the case work requires, where two carrier disks are used, the user to view different windows and, in addition, the space available around the carrier disk means that the size of the numbers would be small.

A useful method is to employ a display with separate units and tens indication, driven such that the tens display index one number as the unit display index from 9 to 0. This allows larger numbers to be used within the same casework. The two disks may be provided concentrically one within the other and, in some cases, coaxially with the axis of the device, for instance on shaft 68 illustrated in FIG. 11. The displayed units and tens are visible through the window 10 illustrated in FIG. 1(a). The indexing of the device, in addition to moving the next pocket insert 26 into alignment with the prodgers, actuates the counter mechanism 140 that provides a visual indication to the user of how many unitary doses 28 have been dispensed and/or how may unitary doses 28 remain unused. An example of a suitable counter mechanism 140, implementing a unit and tens counter driven by a driving gear meshing with one of the gears 78 of the Geneva wheel 100 of the indexing mechanism, is described in WO-A-2005/002654. The driving gear and the unit and tens counters are adapted to index a tens display of the counter display 9 of one number as a unit display of the counter display 9 is indexed from 9 to 0.

In one embodiment, the display counts down to zero, but the tens disk is not provided with a "0". Instead, it is provided with an indicator, for instance a symbol or color light to indicate to the user that the device is nearing the end of its functional life.

One embodiment uses another Geneva and gear arrangement that is driven from the movement of the carrier disks. It is indicated that a single counter is increment initially by the motion of the first carrier disk and subsequently by the motion of the second carrier disk such that the fact that the device contains two carrier disks is not apparent to the user.

After the last dose has been used, the remaining doses display will read 0 indicating that the device is empty to the user. However, if the user does not look at the display, they may actuate the device again when desiring further doses. A suitable device will provide some positive feedback to the user, as it is being actuated, that it is empty.

This feedback can be in the form that priming lever 8 cannot be moved to its operating position with the level of force normally used. This tactile feedback provides a lockout feature.

A useful method of achieving this with the two disk device is to arrange that after the last dose has been used, the second disk indexes such that it has no pocket under the prodger. At this point, two prodger members 76 both face surfaces of the disks without pockets. Thus as priming lever 8 is moved, neither prodger member 76 can move onto a disk and the resulting force on prodger members 76 is transmitted back through the drive mechanism to priming lever 8 and hence to the user.

While the user may be able to apply sufficient force to move priming lever 8 through to its home position, this will only be possible by forcing the disks to separate against the constraint of the casework. The force required to do this can be made sufficiently greater that the normal actuation force as to be obvious to the user.

Inhaling devices can be and are sometimes misused. For example, where the user did not push the priming lever substantially to the end of the stroke, returning it from this point will advance the disk to the next dose and decrement the counter, even though a dose has not been made available for inhalation. This happens approximately one third of the way through the stroke, well before the pocket is opened.

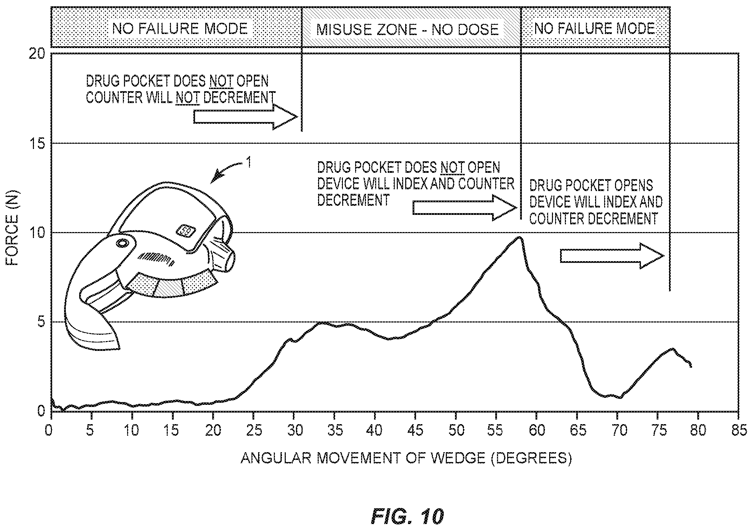

In the event of misuse, the actuation step cannot be easily paused in the misuse region. If the priming lever is returned before the pocket is opened, the device will index and count despite no dose being delivered. This behavior can be repeated for multiple actuation cycles. As a result, the device has a rising force throughout the misuse region as illustrated in Figure 10.

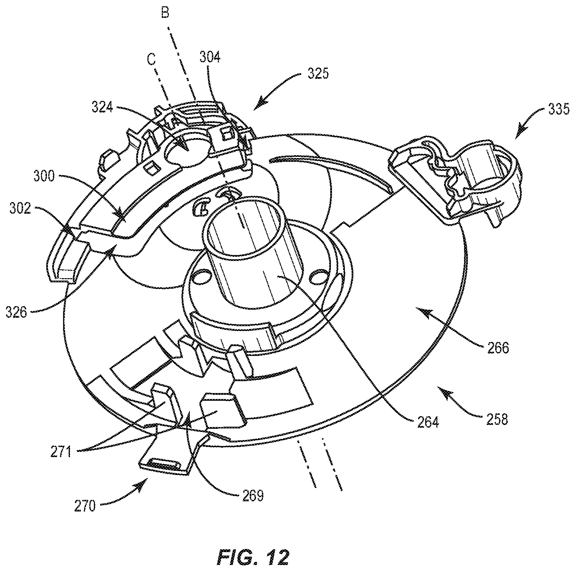

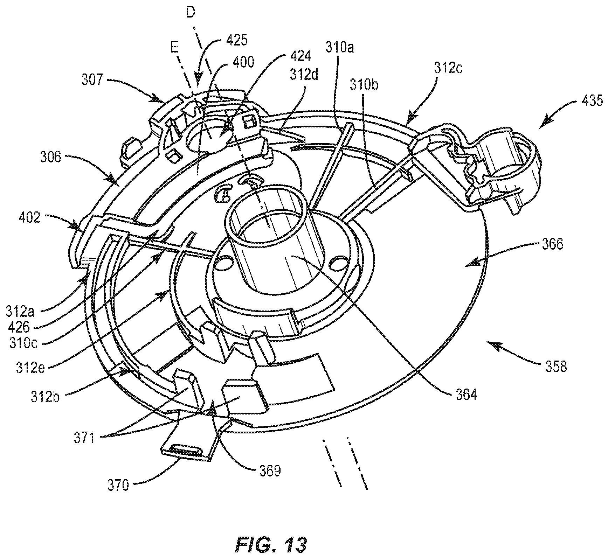

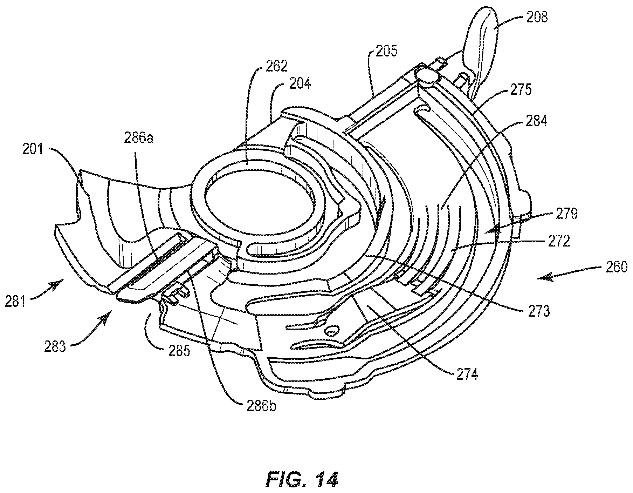

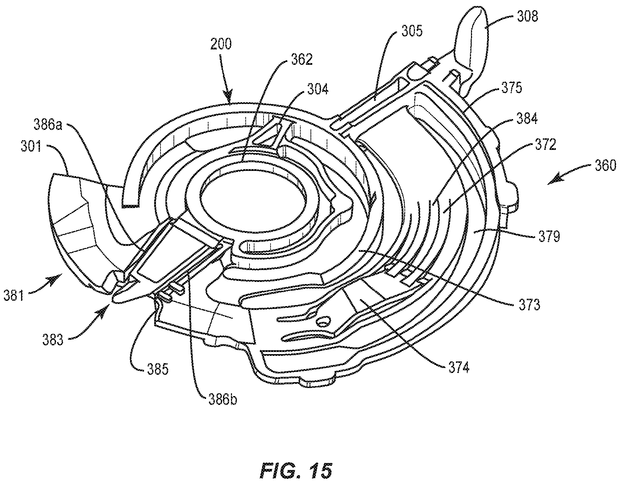

To reduce or even eliminate the potential for misuse of an inhaler by not actuating the priming lever fully or through a nonconformance with instructions for use, the geometry of the chassis and the priming member of the actuating mechanism can be improved. For example, other embodiments for the chassis of the actuating mechanism of an inhaler are shown in FIGS. 12 and 13. Other embodiments of the priming member of the actuating mechanism are shown in FIGS. 14 and 15.

FIG. 12 illustrates chassis assembly 258 which is disk-shaped, contains hollow pivot shaft 264, peripherally disposed casing 335 and clip 325. Hollow pivot shaft 264 can be fitted on shaft 68 of casing 2b shown in FIG. 11. To complete the actuating mechanism of an inhaler, shaft 264 is adapted to receive a priming member. Chassis 266 is also provided with radial extension 270 which interacts with an inner portion of housing 2 so as to rotationally fix chassis 266. Chassis 266 comprises guide members 271 which extend axially and define radial aperture 269 between them. Aperture 269 allows prodgers (not shown) to extend through chassis 266 towards a corresponding carrier 12. Formed as one piece with chassis 266, peripherally disposed casing 335 is adapted to support a component of a changeover mechanism, an important feature in some inhalers. Chassis 266 further comprises clip 325 which contains aperture 324 for receiving the Geneva index mechanism illustrated in FIG. 9.

As distinct from chassis assembly 58 shown in FIG. 9, chassis assembly 258 of FIG. 12 comprises shelf 300 disposed adjacently to aperture 324 of clip 325. Shelf 300 defines aperture 326 bound on one end by supporting wall 302 and at the other end by recessed connector 304.

In some applications, chassis incorporating a shelf element may distort the chassis structure by rendering the Geneva indexing mechanism too flexible. To overcome potential distortions additional supporting structures are provided as illustrated in FIG. 13.

FIG. 13 illustrates another embodiment of a chassis assembly 358 which is disk-shaped, contains hollow pivot shaft 364, peripherally disposed casing 435 and clip 425. Hollow pivot shaft 364 can be fitted on shaft 68 of casing 2b shown in FIG. 11. To complete the actuating mechanism of an inhaler, shaft 364 is adapted to receive a priming member. Chassis 366 is also provided with radial extension 370 which interacts with an inner portion of housing 2 so as to rotationally fix chassis 366. Chassis 366 comprises guide members 371 which extend axially and define radial aperture 369 between them. Aperture 369 allows prodgers (not shown) to extend through chassis 366 towards a corresponding carrier 12. Formed as one piece with chassis 366, peripherally disposed casing 435 is adapted to support a component of a changeover mechanism, an important feature in some inhalers. Chassis 366 further comprises clip 425 which contains aperture 424 for receiving the Geneva index mechanism illustrated in FIG. 9.

As distinct from chassis assembly 58 shown in FIG. 9, chassis assembly 358 of FIG. 13 comprises shelf 400 disposed adjacently to aperture 424 of clip 425. Shelf 400 defines aperture 426 bound on one end by supporting wall 402 and at the other end by buttress support 312d. Other buttresses 312a, 312b, 312c and 312e, generally circumferentially disposed improve the mechanical stability of chassis 366 and the Geneva index mechanism placed in aperture 424. Chassis 366 may also incorporate a plurality of radially disposed ribs, 310a, 310b, and 310c, also meant to reinforce the mechanical stability of chassis 366. Peripherally disposed low step 306 and high step 307 located adjacent clip 425 and opposite shelf 400 can also be incorporated in chassis 366 to improve the molding process.

The actuating mechanism of inhaler 1 can include other embodiments of the priming member as illustrated in FIGS. 14 and 15. FIG. 14 illustrates priming member 260. Formed of a disk-shaped plate molded in plastic, priming member 260 has a central pivot opening 262 by which it is rotatably supported on pivot shaft 264 of the chassis 266, for example. Priming member 260 includes an elongate cam member 272 formed on priming member 260 and separated from the remaining part of priming member 260 by elongate cam openings 279 through which guide members 271 of chassis 266 can extend. Elongate cam member 272 extends in a circumferential direction and presents a profile adapted to provide a limited amount of flexibility. Central cam surface 274 is provided on each of two opposite sides of cam member 272. Lateral cam surfaces 273, 275 extend on either side of priming member 260, in circumferential directions along elongate cam openings 279, opposite elongate cam member 272. Priming member 260 includes elongate cam openings 279 on either side of cam member 272 through which arms of the prodgers (not shown) can extend. As illustrated in FIG. 14, cam surfaces 274 are provided with one or more grooves or channels 284. To provide additional stability, priming member 260 includes a tapered connector 204 joining lateral cam surface 273 and central pivot opening 262 and raised connector 205 positioned adjacent priming lever 208.

Rotary priming member 260 incorporates many of the functional elements described previously within a single molded component. It includes priming lever 208, cam member 274, prodgers (not shown), and flexible cantilever blade 283, as well as being the driving member for indexing Geneva wheel 100.

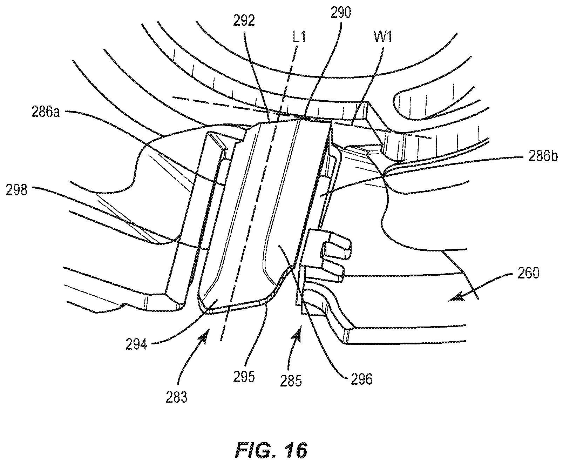

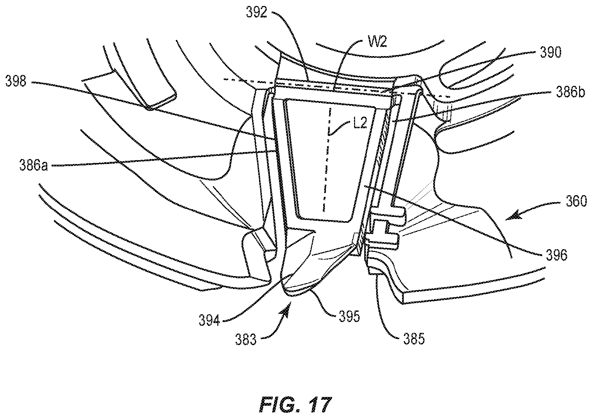

Driving member 281 is provided with a leading portion 201 which contains a cut out to facilitate assembly, flexible cantilever blade 283 which can travel over and under chassis shelf 300 or 400 illustrated in FIGS. 12 or 13, respectively, with a trailing edge 285 arranged in sequence. Cantilever blade 283 is flexibly attached to priming member 260 through a hinge flexure allowing it to move in an upward and downward direction and has two openings on either side, 286a and 286b. Additional details for the structure of cantilever blade 283 are illustrated in FIG. 16. Cantilever blade 283 of FIG. 16 has a proximal portion 292, a distal portion 295 and a tip 294 disposed at distal portion 295. Cantilever blade 283 comprises a longitudinal axis L1 extending from proximal portion 292 to distal portion 295 and a flexible portion 290 which extends transverse to longitudinal axis L1 following a transverse axis W1. Cantilever blade 283 has a first surface 296 and a second surface 298. Both, first surface 296 and second surface 298 extend along longitudinal axis L1. First surface 296 is disposed opposite second surface 298 such that a first gap 286a is formed between priming member 260 and first surface 296 and a second gap 286b is formed between priming member 260 and second surface 298. The body of cantilever blade 283 can have a generally rectangular or a tapered body shape as illustrated in FIGS. 15 and 17.

Another embodiment of the priming member of the actuating mechanism is illustrated in FIG. 15. Priming member 360 has a central pivot opening 362 by which it is rotatably supported on pivot shaft 364 of chassis 366, for example. Priming member 360 may be formed of a disk-shaped plate molded in plastic. Priming member 360 includes an elongate cam member 372 formed on priming member 360 and separated from the remaining part of priming member 360 by elongate cam openings 379 through which guide members 371 of chassis 366 can extend. Elongated cam member 372 extends in a circumferential direction and presents a profile adapted to provide a limited amount of flexibility. Central cam surface 374 is provided on each of two opposite sides of cam member 372. Lateral cam surfaces 373, 375 extend on either side of priming member 360, in circumferential directions along elongate cam openings 379, opposite elongate cam member 372. Priming member 360 includes elongate cam openings 379 on either side of cam member 372 through which arms of the prodgers (not shown) can extend. As illustrated in FIG. 15, central cam surfaces 374 are provided with one or more grooves or channels 384. To provide additional stability, and avoid unwanted flex or twist, priming member 360 comprises a circumferential rib 200 which connects lateral cam surface 373 and leading portion 301 of driving member 381. Further, to improve the robustness of priming member 360, leading portion 301 does not contain a cutout portion as in the embodiment of FIG. 14. In addition, the connectors adjacent priming lever 308 and joining lateral cam surface 373 to central pivot opening 362 have been cored to form recessed connector 304 and recessed tapered connector 305. Coring of these connectors served to control potential distortion of the priming member.

Rotary priming member 360 incorporates many of the functional elements described previously within a single molded component. It includes priming lever 308, cam member 374, prodgers (not shown), and flexible tapered cantilever blade 383, as well as being the driving member for indexing Geneva wheel 100. Flexible cantilever blade 383 which can travel over and under chassis shelf 300 or 400 illustrated in FIGS. 12 or 13, respectively, is part of leading portion 301 which also contains a trailing edge 385 arranged in sequence. Cantilever blade 383 is flexibly attached to priming member 360 through a hinge flexure allowing it to move in an upward and downward direction and has two openings on either side, 386a and 386b. Additional details for the structure of tapered cantilever blade 383 are illustrated in FIG. 17. Cantilever blade 383 of FIG. 17 has a proximal portion 392, a distal portion 395 and a tip 394 disposed at distal portion 395. Cantilever blade 383 comprises a longitudinal axis L2 extending from proximal portion 392 to distal portion 395 and a flexible portion 390 which extends transverse to longitudinal axis L2 following a transverse axis W2. Tapered cantilever blade 383 has a first surface 396 and a second surface 398. Both, first surface 396 and second surface 398 extend along longitudinal axis L2. First surface 396 is disposed opposite second surface 398 such that a first gap 386a is formed between priming member 360 and first surface 396 and a second gap 386b is formed between priming member 360 and second surface 398. To optimize the interaction with the chassis shelf, tip 394 contains angled chamfers.

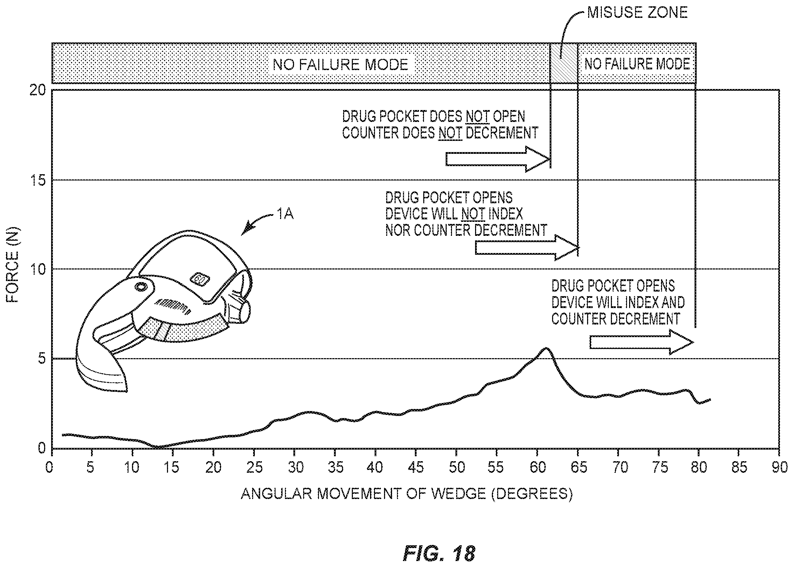

The misuse of the actuating mechanism comprising the chassis and priming members illustrated in FIGS. 12-17 may form a misuse region as illustrated in FIG. 18. In the misuse region the actuation step cannot be easily paused. If the priming lever is returned before the pocket is opened, the priming member would not have reached the end of the chassis shelf and would return on top of the shelf. The device would neither index nor count and the counter would temporarily be delayed and show one index behind the doses taken. On the next actuation, however, the same pocket will be prodged again, no dose would be delivered, but the device would index and count normally and the counter would now correctly represent the doses taken. Changes in the structure of the chassis and the priming member as illustrated in the embodiments of FIGS. 12-17 result in significantly reducing or even potentially eliminating the misuse zone as illustrated in FIG. 18. While, in some embodiments, the priming lever can travel one third of the way in a misuse zone, in the embodiments of FIGS. 12-17, the priming lever travels less than about 5% in the misuse zone. As a result, in these embodiments, as illustrated in FIG. 18, there is a substantial reduction in the force profile through the misuse region. In the embodiments of FIGS. 12-17, the impact of misuse is no longer critical and while the counter is temporarily out of synchronization with doses delivered, it corrects on the next use.

On the actuation stroke, the cantilever blade passes over the shelf on the chassis, bypassing the Geneva indexing mechanism, and the end of the shelf is synchronized with dose opening. If the priming lever is not pushed substantially to the end of stroke, the cantilever blade will return on the top of the shelf without indexing the disk or the counter, which will correctly indicate that no dose has been taken. During a correct actuation (to the end of a stroke) the cantilever blade will drop off the end of the shelf just after the dose opens. When the priming lever is returned, the cantilever blade then passes under the chassis shelf where it can engage with the Geneva indexing mechanism to advance the disk insert to the next dose and decrement the counter.

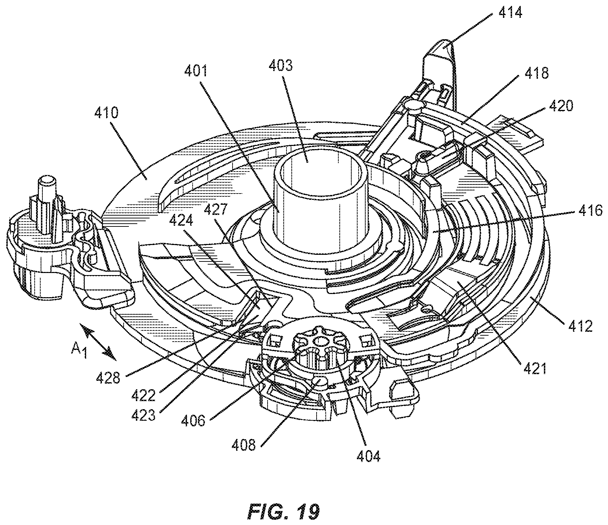

FIG. 19 illustrates an embodiment of a chassis 410, which is disk-shaped coupled to the priming member 412, which is also disk-shaped, the chassis contains hollow pivot shaft 401 and the priming member is attached to the pivot shaft around opening 403. The priming member can rotate on axis A1 about the chassis on delivery of the dose of the medicine and the counter will indicate a dose is delivered. A Geneva wheel 404 is provided and attached to the chassis. The Geneva wheel also includes gears 406 coaxial with peg wheel 408 and is adapted to cooperate respectively with the coupling portions and supports.

The priming member incorporates many of the functional elements described previously. It includes priming lever 414, cam member 421, prodger 420, and cantilever blade 422, as well as being the driving member for indexing the Geneva wheel.

The priming member includes a cantilever blade configured to engage a chassis of the inhaler, the cantilever blade 422 has a proximal portion 423, a body 424 and a distal portion, the body being disposed between the proximal portion and the distal portion of the cantilever blade and the cantilever blade having a flexible portion 427 extending with the proximal portion of the cantilever blade to allow movement of the cantilever blade relative to the chassis. The cantilever blade is angled in a direction toward the chassis (e.g., in a downward direction) at about 5, 10, 15, 20, 25, 30, 35 to about 40 degrees. The flexible portion is a hinge flexure allowing the cantilever blade to move in an upward and downward direction relative to the chassis. The cantilever blade allows a uniform delivery of the medication and the accurate counting of the dose of the medication.

Adjacent the cantilever blade is a first gap 428 that is formed between the priming member and the cantilever blade. This gap, among other things, allows space for vertical movement of the cantilever blade relative to the chassis and/or priming member.

**In** the illustrated embodiment, the prodger 420 is mounted on the priming member, and lateral cam surfaces 416 and 418 are arranged on the priming member to provide stability to the inhaler.

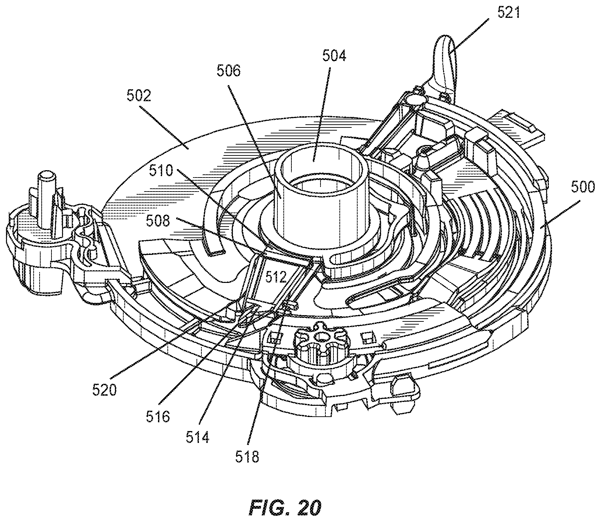

FIG. 20 illustrates an embodiment of a chassis 502, which is disk-shaped coupled to the priming member 500, which is also disk-shaped, the chassis contains hollow pivot shaft 506 and the priming member is attached to the pivot shaft around opening 504. The priming member can rotate about the chassis on delivery of the dose of the medicine and the counter will indicate a dose is delivered. A Geneva wheel is provided and attached to the chassis.

The priming member incorporates many of the functional elements described previously. It includes priming lever 521 and cantilever blade 512, as well as being the driving member for indexing the Geneva wheel.

The priming member includes a cantilever blade 512 configured to engage a chassis of the inhaler, the cantilever blade having a proximal portion 510, a body and a distal portion 514, the body being disposed between the proximal portion and the distal portion of the cantilever blade and the cantilever blade having a flexible portion 508 extending with the proximal portion of the cantilever blade to allow movement of the cantilever blade relative to the chassis. The cantilever blade is shown as a triangular shape that is angled in a direction toward the chassis (e.g., in a downward direction) at about 5, 10, 15, 20, 25, 30, 35 to about 40 degrees. The flexible portion can be a hinge allowing the cantilever blade to move in an upward and downward direction relative to the chassis. The cantilever blade comprises a tip 516 that can be tapered and angled as shown. The cantilever blade allows a uniform delivery of the medication and the accurate counting of the dose of the medication.

Adjacent the cantilever blade is a first gap 520 and a second gap 518 that is formed between the priming member and the cantilever blade. These gaps, among other things, allow space for vertical movement of the cantilever blade relative to the chassis and/or priming member. It will be understood that the flexible portion 508 may be made from the same material as the priming member. It can be designed with a hinge, in some embodiments, a joint to allow vertical movement of the cantilever blade relative to the priming member and/or chassis. First gap 520 and second gap 518 can be parallel or substantially parallel to each other and extend longitudinally with at least a portion of the cantilever blade 512. In some embodiments, the cantilever blade is adapted to pass over a portion of the chassis upon actuation of the inhaler and under a portion of the chassis upon indexing of the inhaler.

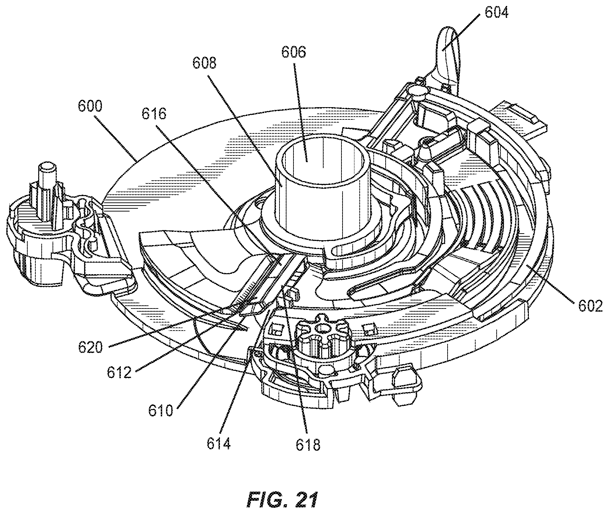

FIG. 21 illustrates an embodiment of a chassis 600, which is disk-shaped coupled to the priming member 602, which is also disk-shaped, the chassis contains hollow pivot shaft 608 and the priming member is attached to the pivot shaft around opening 606. The priming member can rotate about the chassis on delivery of the dose of the medicine and the counter will indicate a dose is delivered. A Geneva wheel is provided and attached to the chassis.

The priming member incorporates many of the functional elements described previously. It includes priming lever 604 and a cantilever blade, as well as being the driving member for indexing the Geneva wheel.

The priming member includes a cantilever blade configured to engage a chassis of the inhaler, the cantilever blade having a proximal portion 616, an angled body 620 and a distal portion 610, the body being disposed between the proximal portion and the distal portion of the cantilever blade and the cantilever blade having a flexible portion extending with the proximal portion of the cantilever blade to allow movement of the cantilever blade relative to the chassis. The cantilever blade is shown as a rectangular shape and has an elevated portion 618 that is angled in a direction away from the chassis (e.g., in an upward direction) at about 5, 10, 15, 20, 25, 30, 35 to about 40 degrees. The flexible portion can be a hinge allowing the cantilever blade to move in an upward and downward direction relative to the chassis. The cantilever blade comprises a tip that can be chamfered as shown. The cantilever blade allows a uniform delivery of the medication and the accurate counting of the dose of the medication.

Adjacent the cantilever blade is a first gap 612 and a second gap 614 that is formed between the priming member and the cantilever blade. These gaps, among other things, allow space for vertical movement of the cantilever blade relative to the chassis and/or priming member. It will be understood that the flexible portion may be made from the same material as the priming member and have a reduced thickness or an increased thickness relative to the rest of the cantilever blade to function as a hinge to allow movement of the remainder of the cantilever blade. It can be designed with a hinge, in some embodiments, a joint to allow vertical movement of the cantilever blade relative to the priming member and/or chassis. First gap 612 and second gap 614 can be parallel or substantially parallel to each other and extend longitudinally with at least a portion of the cantilever blade. In some embodiments, the cantilever blade is adapted to pass over a portion of the chassis upon actuation of the inhaler and under a portion of the chassis upon indexing of the inhaler.

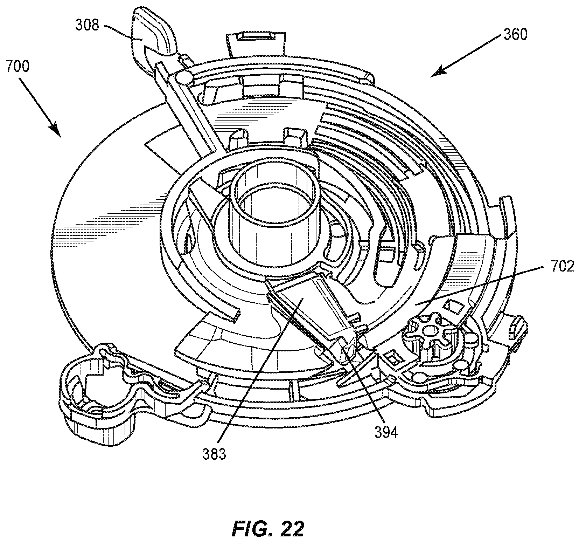

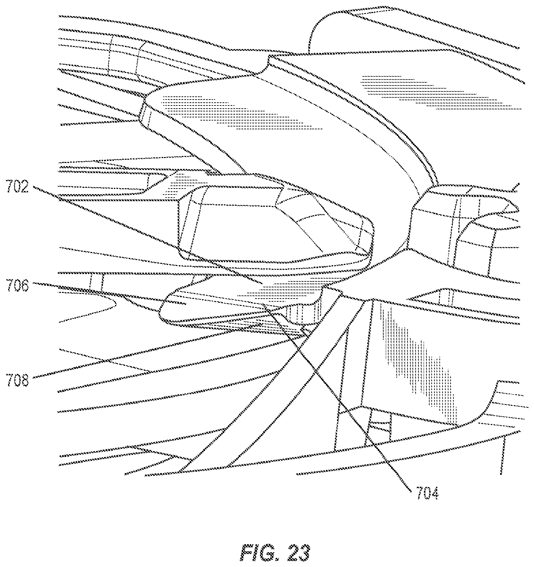

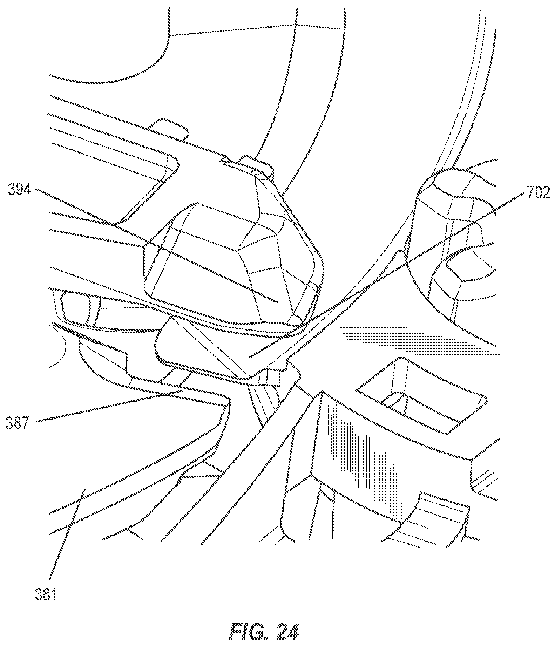

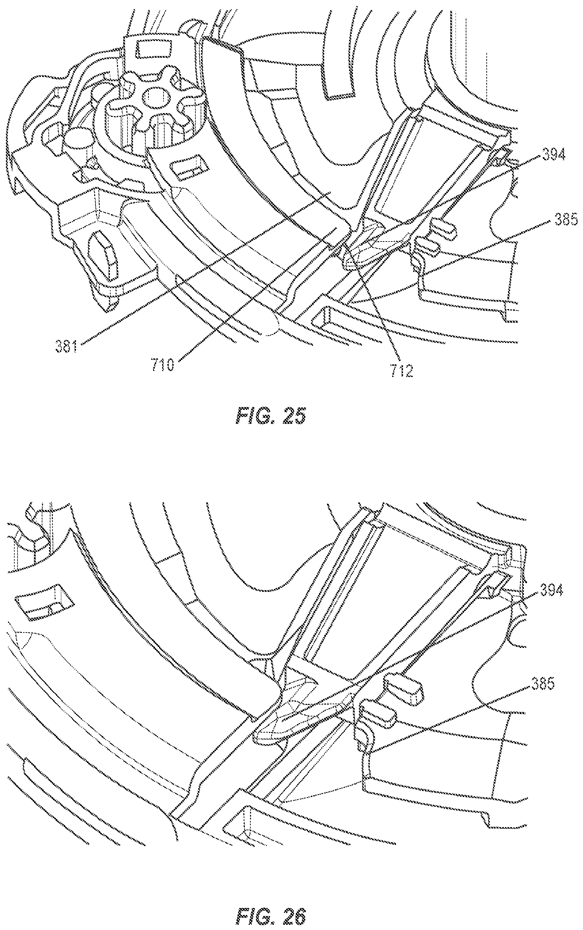

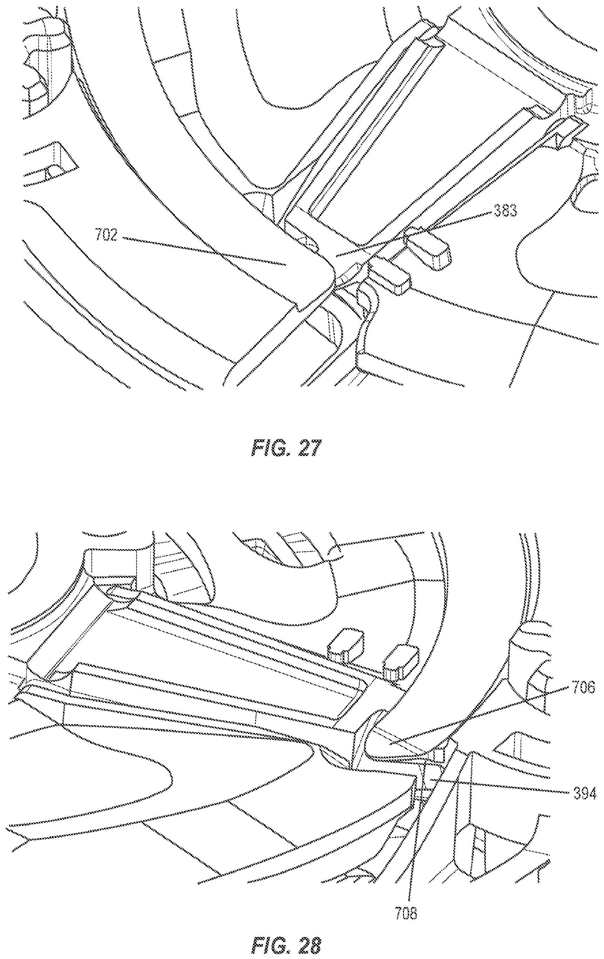

FIGS. 22-28 illustrate an embodiment of a chassis 700, which is disk-shaped coupled to the priming member 360 of FIGS. 15 and 17. The chassis 700 incorporates many of the functional elements described previously. The chassis 700 further includes a chassis shelf 702 having a leading edge 704 and a trailing edge 710. The leading edge 704 includes an upper angled chamfer 706 and a lower angled chamfer 708, as shown in FIG. 23. The angled chamfers 706, 708 are configured to engage with the cantilever tip 394 such that the cantilever tip 394 passes over the chassis shelf 702 during the actuation or prodger stroke (FIGS. 23 and 24) and under the chassis shelf 702 during indexing or counting stroke (FIGS. 25-28). However, adjacent sections of the driving member 381 can pass under the chassis shelf 702. It is to be understood that movement of the cantilever tip 394 over and under the chassis shelf 702 allows the cantilever blade 383 to interact better with the counter. For example, if the priming lever 308 is moved backward, it will not engage the counter until the activation stroke is performed. Therefore, there is a reduced chance of miscounting, or miscounting of doses is eliminated.

The upper angled chamfer 706 is angled to cooperate with the cantilever tip 394 and to direct the cantilever tip 394 upward and over the chassis shelf 702. The lower angled chamfer 708 is angled to cooperate with an angled end 387 of driving member 381 and to direct the cantilever tip 394 under the chassis shelf 702. In some embodiments, the angled chamfers 706, 708 and/or the angled end 387 of driving member 381 can be angled from about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 to about 40 degrees relative planar chassis shelf.

As discussed above, to optimize the interaction with a chassis shelf, cantilever tip 394 contains angled chamfers that can have an angle from about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 to about 40 degrees relative planar chassis shelf. Cantilever tip 394 is also angled to optimize interaction with upper angled chamfer 706.

Both cantilever blade 383 and driving member 381 pass under the chassis shelf 702 on actuation, so that the trailing edge 710 of the chassis shelf 702 has only one chamfer 712 facing downwards (FIG. 25), and a surface of the cantilever tip 394 is angled to interact with chamfer 712. Chamfer 712 is also configured to engage with trailing edge 385 of priming member 360.

It will be apparent to those skilled in the art that various modifications and variations can be made to various embodiments described herein without departing from the scope of the teachings herein. Thus, it is intended that various embodiments cover other modifications and variations of various embodiments within the scope of the present teachings.

The scope of the present invention is defined by the claims that follow.

## Claims

1. A priming member (360) for an inhaler (1) for inhaling a dose of a powder, the priming member comprising a cantilever blade (383) configured to engage a chassis (266) of the inhaler, the cantilever blade (383) having a proximal portion (392), a body and a distal portion (395), the body being disposed between the proximal portion and the distal portion of the cantilever blade (383), and **characterized by** the cantilever blade having a flexible portion (390) extending with the proximal portion of the cantilever blade to allow movement of the cantilever blade relative to the chassis, wherein the flexible portion (390) is a hinge flexure allowing the cantilever blade to move in an upward and downward direction relative to the chassis of the inhaler, such that the cantilever blade (383) is configured to pass over a shelf (300) of the chassis (266) upon actuation of the inhaler for operating a dispensing mechanism of the inhaler and under the shelf (300) of the chassis (266) upon indexing of the inhaler for operating an indexing mechanism and a dose counter of the inhaler.

2. The priming member of claim 1, wherein the body of the cantilever blade (383) comprises a longitudinal axis (L2) extending from the proximal portion (392) to the distal portion (395) of the cantilever blade, and the flexible portion (390) extends transverse to the longitudinal axis.

3. The priming member of claim 2, wherein the cantilever blade (383) comprises a first surface (396) and a second surface (398) extending along the longitudinal axis (L2) of the cantilever blade, wherein the first surface is opposite the second surface such that a first gap (386a) is formed between the priming member and the first surface of the cantilever blade and a second gap (386b) is formed between the second surface and the priming member.

4. The priming member of claim 1, wherein the distal portion (395) of the cantilever blade comprises an angled tip (394).

5. The priming member of claim 1, wherein the cantilever blade has a wedge shape or a rectangular shape.

6. The priming member of claim 4, wherein the tip of the cantilever blade is chamfered.

7. An inhaler (1) for inhaling a dose of powder, the inhaler comprising a chassis (266), a mouthpiece (6) for inhaling the dose of powder, and a priming member (360) according to claim 1, the priming member being fluidly coupled to the mouthpiece.

## Patentansprüche

1. Eine Vorbereitungseinrichtung (360) für einen Inhalator (1) zum Inhalieren einer Do-sis eines Pulvers, wobei die Vorbereitungseinrichtung einen Auslegerschenkel (383) umfasst, der dazu eingerichtet ist, in ein Chassis (266) des Inhalators einzugreifen, wobei der Auslegerschenkel (383) einen proximalen Abschnitt (392), einen Körper und einen distalen Abschnitt (395) aufweist, wobei der Körper zwischen dem proxi-malen Abschnitt und dem distalen Abschnitt des Auslegerschenkels (383) angeordnet ist, **dadurch gekennzeichnet, dass**
der Auslegerschenkel einen flexiblen Abschnitt (390) aufweist, der sich mit dem pro-ximalen Abschnitt des Auslegerschenkels erstreckt, um eine Bewegung des Ausleger-schenkels relativ zum Chassis zu ermöglichen, wobei
der flexible Abschnitt (390) ein Biegegelenk ist, das es dem Auslegerschenkel ermög-licht, sich relativ zum Chassis des Inhalators in Aufwärts- und Abwärtsrichtung zu bewegen, so dass der Auslegerschenkel (383) derart eingerichtet ist, dass er bei Betäti-gung des Inhalators zum Ausführen eines Abgabemechanismus des Inhalators über einen Vorsprung (300) des Chassis (266) geführt wird und bei der Schaltung des Inha-lators zum Betätigen einer Schalteinrichtung und eines Dosierzählers des Inhalators unter den Vorsprung (300) des Chassis (266).

2. Die Vorbereitungseinrichtung nach Anspruch 1, wobei der Körper des Auslegerschen-kels (383) eine Längsachse (L2) umfasst, die sich von dem proximalen Abschnitt (392) zu dem distalen Abschnitt des Auslegerschenkels erstreckt, und der flexible Abschnitt (390) sich quer zu der Längsachse erstreckt.

3. Die Vorbereitungseinrichtung nach Anspruch 2, wobei der Auslegerschenkel (383) eine erste Oberfläche (396) und eine zweite Oberfläche (398) umfasst, die sich ent-lang der Längsachse (L2) des Auslegerschenkels erstrecken, wobei die erste Oberflä-che der zweiten Oberfläche gegenüberliegt, so dass ein erster Spalt (386a) zwischen der Vorbereitungseinrichtung und der ersten Oberfläche des Auslegerschenkels und ein zweiter Spalt (386b) zwischen der zweiten Oberfläche und der Vorbereitungsein-richtung gebildet wird.

4. Die Vorbereitungseinrichtung nach Anspruch 1, wobei der distale Abschnitt (395) des Auslegerschenkels eine gewinkelte Spitze (394) umfasst.

5. Die Vorbereitungseinrichtung nach Anspruch 1, wobei der Auslegerschenkel eine Keil-form oder eine rechteckige Form aufweist.

6. Die Vorbereitungseinrichtung nach Anspruch 4, wobei die Spitze des Auslegerschen-kels abgeschrägt ist.

7. Ein Inhalator (1) zum Inhalieren einer Dosis Pulver, wobei der Inhalator ein Chassis (266), ein Mundstück (6) zum Inhalieren der Dosis Pulver und eine Vorbereitungsein-richtung (360) gemäß Anspruch 1 umfasst, wobei die Vorbereitungseinrichtung flui-disch mit dem Mundstück verbunden ist.

## Revendications

1. Élément d'amorçage (360) pour un inhalateur (1) pour inhaler une dose de poudre, l'élément d'amorçage comprenant une lame cantilever (383) configurée pour insérer un châssis (266) de l'inhalateur, la lame cantilever (383) ayant une portion proximale (392), un corps et une portion distale (395), le corps étant disposé entre la portion proximale et la portion distale de la lame cantilever (383), et **caractérisé en ce que** la lame cantilever a une portion flexible (390) s'étendant avec la portion proximale de la lame cantilever pour permettre le mouvement de la lame cantilever par rapport au châssis,
dans lequel la portion flexible (390) est une charnière permettant à la lame cantilever de se déplacer vers le haut et vers le bas par rapport au châssis de l'inhalateur, de sorte que la lame cantilever (383) est configurée pour passer au-dessus d'une clayette (300) du châssis (266) lors du déclenchement de l'inhalateur pour actionner un mécanisme de distribution de l'inhalateur et au-dessous de la clayette (300) du châssis (266) lors de l'indexation de l'inhalateur pour actionner un mécanisme d'indexation et un compteur de doses de l'inhalateur.

2. Élément d'amorçage selon la revendication 1, dans lequel le corps de la lame cantilever (383) comprend un axe longitudinal (L2) s'étendant depuis la portion proximale (392) jusqu'à la portion distale (395) de la lame cantilever, et la portion flexible (390) s'étend perpendiculairement à l'axe longitudinal.

3. Élément d'amorçage selon la revendication 2, dans lequel la lame cantilever (383) comprend une première surface (396) et une seconde surface (398) s'étendant le long de l'axe longitudinal (L2) de la lame cantilever, dans lequel la première surface est opposée à la seconde surface de sorte qu'un premier espace (386a) est formé entre l'élément d'amorçage et la première surface de la lame cantilever et un second espace (386b) est formé entre la seconde surface et l'élément d'amorçage.

4. Élément d'amorçage selon la revendication 1, dans lequel la portion distale (395) de la lame cantilever comprend une pointe coudée (394).

5. Élément d'amorçage selon la revendication 1, dans lequel la lame cantilever a une forme triangulaire ou une forme rectangulaire.

6. Élément d'amorçage selon la revendication 4, dans lequel la pointe de la lame cantilever est chanfreinée.

7. Inhalateur (1) pour inhaler une dose de poudre, l'inhalateur comprenant un châssis (266), un embout buccal (6) pour inhaler la dose de poudre, et un élément d'amorçage (360) selon la revendication 1, l'élément d'amorçage étant fluidiquement raccordé à l'embout buccal.
